# EUROPEAN PATENT APPLICATION

(11) **EP 4 815 062 A1**
(43) Date of publication of application: **30.09.2026**
(21) Application number: 24894185.8
(22) Date of filing: 20.11.2024
(51) Int. Cl.: H01M 4/60, C07D 241/46

(54) **ACTIVE SUBSTANCE FOR SECONDARY BATTERY, ELECTRODE FOR SECONDARY BATTERY, SECONDARY BATTERY, AND FLIGHT VEHICLE**

(30) Priority: 24.11.2023 JP 2023199018
(71) Applicant: Softbank Corp., Tokyo 105-7529 (JP)
(72) Inventor: MIYAKAWA Shuntaro, Tokyo 105-7529 (JP); SAITO Takaya, Tokyo 105-7529 (JP); SAKATA Taisei, Tokyo 105-7529 (JP); YAO Masaru, Tsukuba-shi, Ibaraki 305-8560 (JP); UCHIDA Satoshi, Tsukuba-shi, Ibaraki 305-8560 (JP); TAKEICHI Nobuhiko, Tsukuba-shi, Ibaraki 305-8560 (JP); KATO Minami, Tsukuba-shi, Ibaraki 305-8560 (JP); ANDO Hisanori, Tsukuba-shi, Ibaraki 305-8560 (JP)
(74) Representative: Dilg, Haeusler, Schindelmann Patentanwaltsgesellschaft mbH
(86) International application number: PCT/JP2024/041187
(87) International publication number: WO 2025/110194

(57) **Abstract**

Provided is a secondary battery active material used as an active material for a secondary battery, the active material including at least any compound represented by General Formula (1), General Formula (2), General Formula (3), and General Formula (4), each including an oligomer in which a plurality of phenazine structures are bonded, or a salt thereof. Provided is a secondary battery comprising a positive electrode active material layer including a positive electrode active material, a negative electrode active material layer including a negative electrode active material, and an electrolyte, wherein the positive electrode active material or the negative electrode active material contains the above secondary battery active material.

## Description

### TECHNICAL FIELD

The present invention relates to a secondary battery active material, a secondary battery electrode, a secondary battery, and a flight vehicle.

### BACKGROUND ART

Patent Document 1 discloses, as an organic material exhibiting redox activity, the electrode active material for a nonaqueous secondary battery, the electrode active material being composed of a compound in which a naphthazarin skeleton is condensed with a non-conjugated ring such as a dithiin ring.

### RELATED ART DOCUMENTS

### PATENT DOCUMENTS

Patent Document 1: Japanese Patent Application Publication No. 2018-085243

### SUMMARY OF INVENTION

In a first aspect of the present invention, there is provided the secondary battery active material used as the active material for the secondary battery. The above active material includes at least one compound represented by the following General Formula (1), General Formula (2), General Formula (3), and General Formula (4), or a salt thereof.

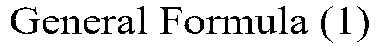

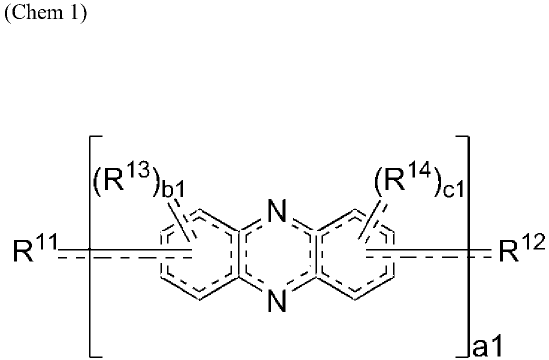

In General Formula (1), R¹¹ at a terminal portion, R¹² at a terminal portion, R¹³, and R¹⁴ may each independently indicate an oxygen atom, a group represented by -OM, a hydrogen atom, or an organic group. M may indicate a hydrogen atom, or a monovalent or divalent metal atom. When M is the divalent metal atom, two adjacent ones of R¹¹ to R¹⁴ may be linked to each other to form a ring represented by -OMO-. A double line indicated by a solid line and a dashed line may indicate a single bond or a double bond. a1 may be an integer of 2 to 10. b1 and c1 may each independently be an integer of 1 to 3. When b1 and c1 are 2 or 3, two or three R¹³ and R¹⁴ may each be the same as or different from each other, and at least four of R¹³ and R¹⁴ may be the oxygen atom or a group represented by -OM.

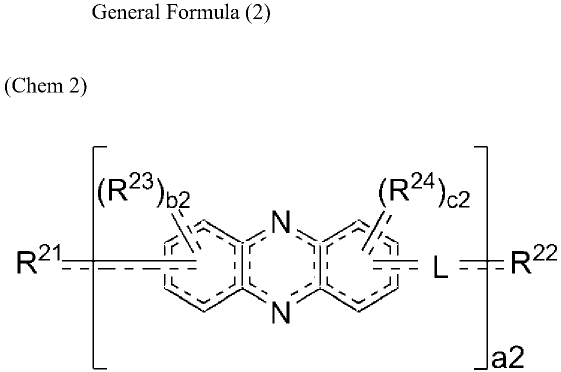

In General Formula (2), R²¹ at the terminal portion, R²² at the terminal portion, R²³, and R²⁴ may each independently represent an oxygen atom, a group represented by -OM, a hydrogen atom, or an organic group. M may represent a hydrogen atom, or the monovalent or divalent metal atom. When M is the divalent metal atom, two adjacent ones of R²¹ to R²⁴ may be linked to each other to form a ring represented by -OMO-. L may be a direct bond or the divalent linker that includes an aromatic group, a double bond, or a triple bond. The double line indicated by a solid line and a dashed line may indicate a single bond or a double bond. a2 may be the integer from 2 to 10. b2 and c2 may each independently be an integer from 1 to 3. When b2 and c2 are 2 or 3, the two or three R²³ and R²⁴ may each be the same as or different from each other, and at least four of R²³ and R²⁴ may be an oxygen atom or a group represented by -OM.

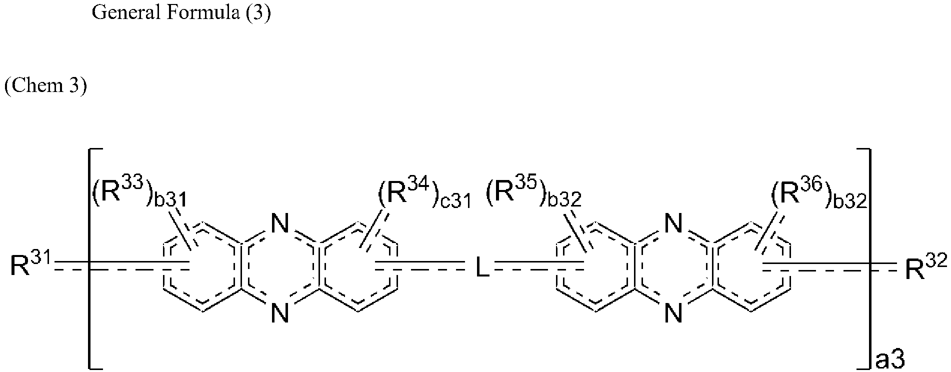

In General Formula (3), terminal R³¹, terminal R³², and R³³ to R³⁶ may each independently represent an oxygen atom, the group represented by -OM, a hydrogen atom, or an organic group. M may represent a hydrogen atom or the monovalent or divalent metal atom. When M is the divalent metal atom, two adjacent ones of R³¹ to R³⁶ may be linked to each other to form a ring represented by -OMO-. L may be a direct bond or the divalent linker including an aromatic group, a double bond, or a triple bond. The double line indicated by a solid line and a dashed line may indicate a single bond or the double bond. a3 may be the integer of 1 to 5. b31, b32, c31, and c32 may each independently be an integer of 1 to 3. When b31, b32, c31, and c32 are 2 or 3, two or three R³³, R³⁴, R³⁵, and R³⁶ may each be the same as or different from each other, and at least four of R³³ and R³⁴ and at least four of R³⁵ and R³⁶ may be the oxygen atom or a group represented by -OM.

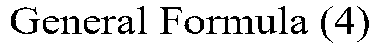

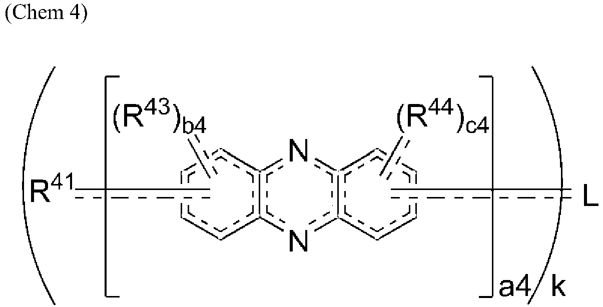

In General Formula (4), terminal R⁴¹, R⁴³, and R⁴⁴ may each independently represent an oxygen atom, the group represented by -OM, a hydrogen atom, or an organic group. M may represent a hydrogen atom or the monovalent or divalent metal atom. When M is the divalent metal atom, two adjacent ones of R⁴¹, R⁴³, and R⁴⁴ may be linked to each other to form a ring represented by -OMO-. L may be a direct bond or the k-valent linker including an aromatic group, a double bond, or a triple bond. A double line indicated by a solid line and a dashed line may indicate a single bond or a double bond. a4 may be the integer of 1 to 5. b4 and c4 may each independently be the integer of 1 to 3. When b4 and c4 are 2 or 3, the two or three R⁴³ and R⁴⁴ may each be the same as or different from each other, and at least four of R⁴³ and R⁴⁴ may be the oxygen atom or a group represented by -OM. k may be an integer of 2 or more.

The above compound may be a compound represented by the following General Formula (5) to General Formula (8).

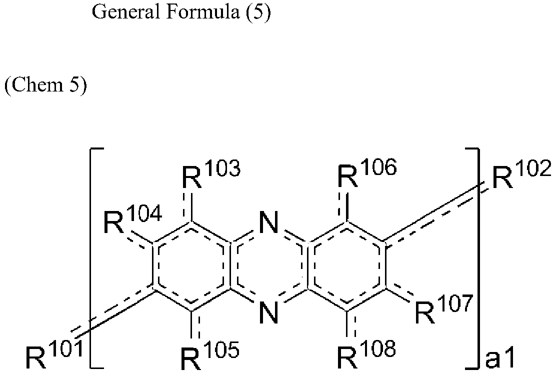

In General Formula (5), terminal R¹⁰¹, terminal R¹⁰², and R¹⁰³ to R¹⁰⁸ may each independently represent an oxygen atom, the group represented by -OM, a hydrogen atom, or an organic group. M may represent a hydrogen atom or the monovalent or divalent metal atom. When M is the divalent metal atom, two adjacent R¹⁰¹ to R¹⁰⁸ may be linked to each other to form a ring represented by -OMO-. The double line indicated by a solid line and a dashed line may represent a single bond or the double bond. At least four of R¹⁰³ to R¹⁰⁸ may be the oxygen atom or a group represented by -OM. a1 may be the integer of 2 to 10.

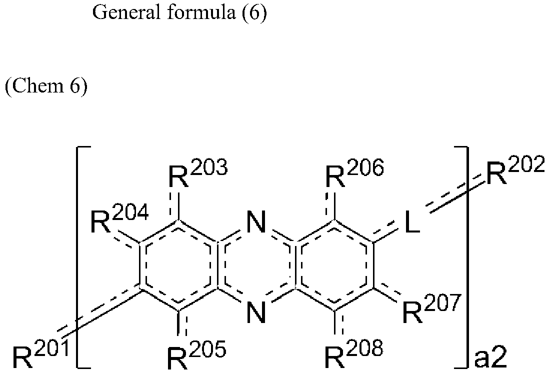

In General Formula (6), terminal R²⁰¹, terminal R²⁰², and R²⁰³ to R²⁰⁸ may each independently represent an oxygen atom, the group represented by -OM, a hydrogen atom, or an organic group. M may represent a hydrogen atom, or the monovalent or divalent metal atom. When M is the divalent metal atom, two adjacent ones of R²⁰¹ to R²⁰⁸ may be linked to each other to form a ring represented by -OMO-. L may be a direct bond or the divalent linker including an aromatic group, a double bond, or a triple bond. The double line indicated by a solid line and a dashed line may represent a single bond or the double bond. At least four of R²⁰³ to R²⁰⁸ may be the oxygen atom or a group represented by -OM. a2 may be the integer from 2 to 10.

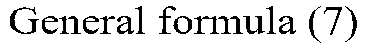

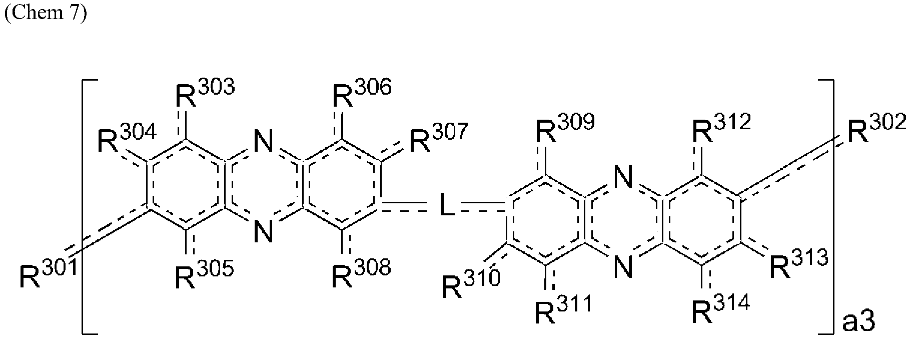

In General Formula (7), terminal R³⁰¹, terminal R³⁰², and R³⁰³ to R³¹⁴ may each independently represent an oxygen atom, the group represented by -OM, a hydrogen atom, or an organic group. M may represent a hydrogen atom, or the monovalent or divalent metal atom. When M is the divalent metal atom, two adjacent ones of R³⁰¹ to R³¹⁴ may be linked to each other to form a ring represented by -OMO-. L may be a direct bond or the divalent linker including an aromatic group, a double bond, or a triple bond. A double line indicated by a solid line and a dashed line may represent a single bond or the double bond. At least four of R³⁰³ to R³⁰⁸ and at least four of R³⁰⁹ to R³¹⁴ may be the oxygen atom or a group represented by -OM. a3 may be the integer from 1 to 5.

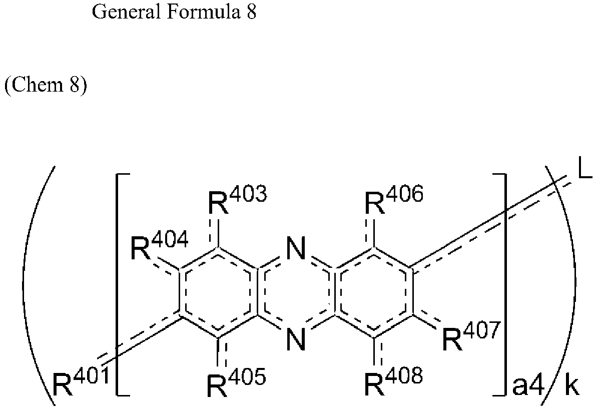

In General Formula (8), terminal R⁴⁰¹ and R⁴⁰³ to R⁴⁰⁸ may each independently represent an oxygen atom, the group represented by -OM, a hydrogen atom, or an organic group. M may represent a hydrogen atom, or the monovalent or divalent metal atom. When M is the divalent metal atom, two adjacent R⁴⁰¹ and R⁴⁰³ to R⁴⁰⁸ may be linked to each other to form a ring represented by -OMO-. L may be a direct bond or the k-valent linker including an aromatic group, a double bond, or a triple bond. The double line indicated by a solid line and a dashed line may indicate a single bond or the double bond. At least four of R⁴⁰³ to R⁴⁰⁸ may be the oxygen atom or a group represented by -OM. a4 may be the integer of 1 to 5. k may be an integer of 2 or more.

The above compound may be a compound represented by the following General Formula (9).

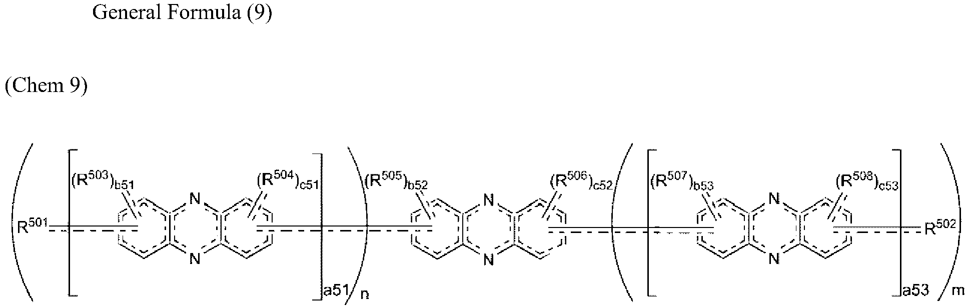

In General Formula (9), terminal R⁵⁰¹, terminal R⁵⁰², and R⁵⁰³ to R⁵⁰⁸ may each independently represent an oxygen atom, the group represented by -OM, a hydrogen atom, or an organic group. M may represent a hydrogen atom, or the monovalent or divalent metal atom. When M is the divalent metal atom, two adjacent R⁵⁰¹ to R⁵⁰⁸ may be linked to each other to form a ring represented by -OMO-. A double line indicated by a solid line and a dashed line may indicate a single bond or the double bond. a51 and a53 may each independently be the integer of 1 to 5. b51, b52, b53, c51, c52, and c53 may each independently be the integer of 1 to 3. When b51, b53, c51, and c53 are 2 or 3, two or three R⁵⁰³, R⁵⁰⁴, R⁵⁰⁷, and R⁵⁰⁸ may each be the same as or different from each other, and at least four of R⁵⁰³ and R⁵⁰⁴ and at least four of R⁵⁰⁷ and R⁵⁰⁸ may be the oxygen atom or the group represented by -OM. n and m may each be an integer of 1 to 3. The sum of n and b52 and the sum of m and c52 may each be 4 or less.

In the above secondary battery active material, the above compound may have symmetry.

In the above secondary battery active material, L may be a direct bond or an organic group including the arylene group, the heterocyclic group, the alkenylene group, or the alkynylene group.

In the above secondary battery active material, the above compound may be at least one of the compounds shown below, or a fully reduced form or a partially reduced form thereof.

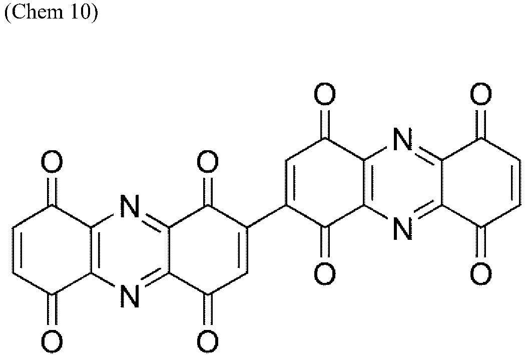

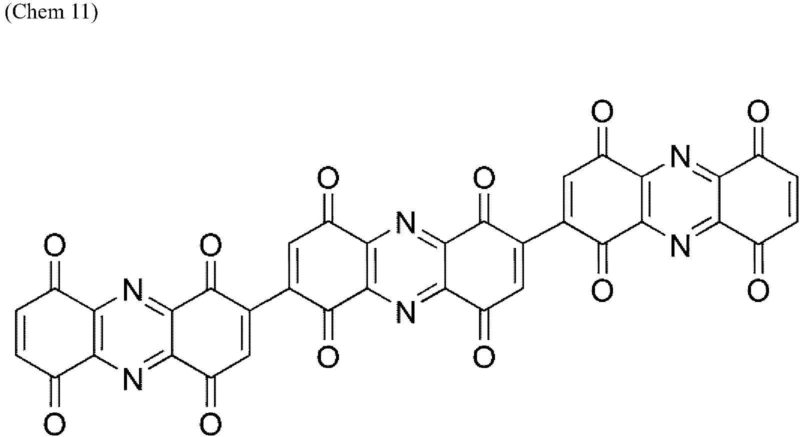

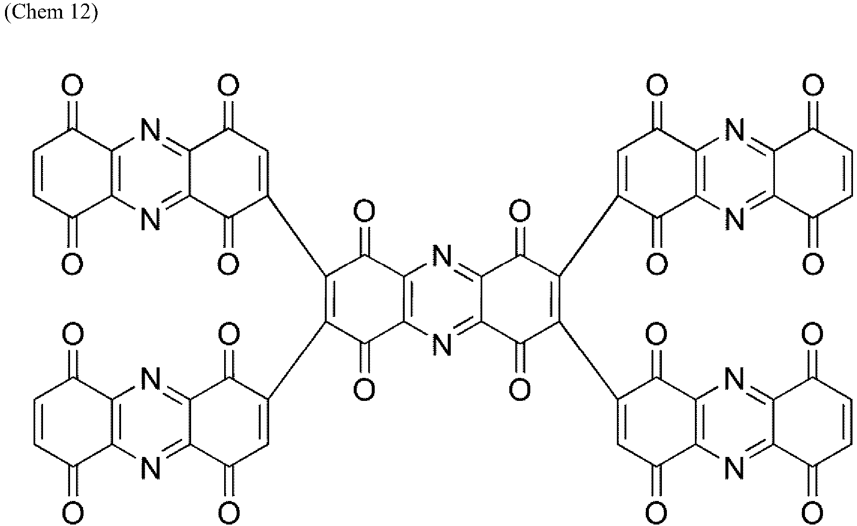

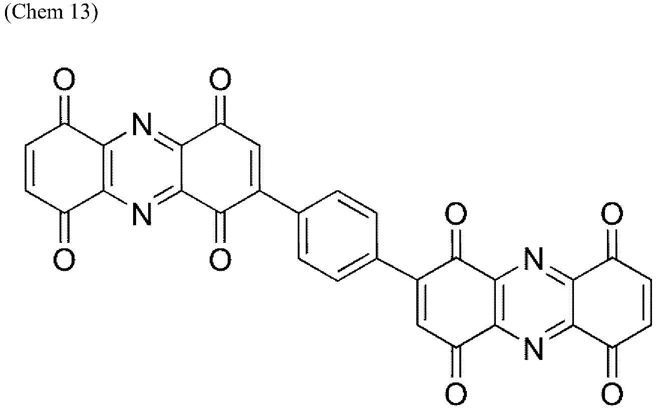

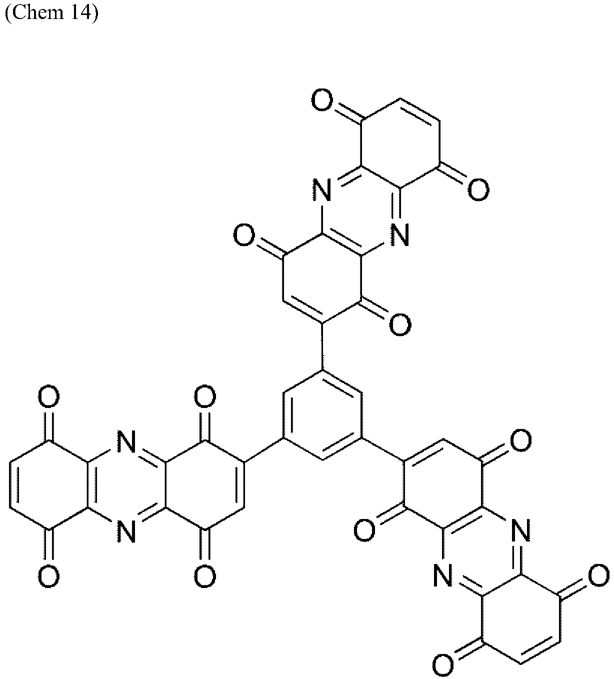

In the above secondary battery active material, M may be H, Li, Na, K, Mg, or Ca.

In a second aspect of the present invention, the secondary battery electrode is provided. The above secondary battery electrode includes the secondary battery active material described above.

In a third aspect of the present invention, the secondary battery is provided. The above secondary battery may have a positive electrode active material layer including the positive electrode active material, a negative electrode active material layer including the negative electrode active material, and the electrolyte. The positive electrode active material or the negative electrode active material may include the secondary battery active material described above.

In the secondary battery described above, the positive electrode active material may include the secondary battery active material described above.

The secondary battery described above may be the nonaqueous secondary battery.

In a fourth aspect of the present invention, a flight vehicle is provided. The flight vehicle described above may include the secondary battery described above, and the propulsive force generator that generates propulsive force by using electrical energy stored in the secondary battery.

Note that the above summary of the invention does not enumerate all the features of the present invention. In addition, sub-combinations of these groups of features may also constitute the invention.

### BRIEF DESCRIPTION OF THE DRAWINGS

FIG. 1 is a diagram schematically illustrating an example of a system configuration of a flight vehicle 100.
FIG. 2 is a diagram schematically illustrating an example of a storage cell 112.
FIG. 3 is a diagram illustrating changes in a capacity retention rate in a charge-discharge test of the secondary battery of this embodiment.
FIG. 4 is a diagram illustrating changes in the capacity retention rate in a charge-discharge test of the secondary battery of this embodiment.

### DESCRIPTION OF EXEMPLARY EMBODIMENTS

Hereinafter, the present invention will be described through embodiments of the invention; however, the following embodiments do not limit the invention according to the claims. In addition, not all combinations of features described in the embodiments are necessarily essential to the means for solving the invention.

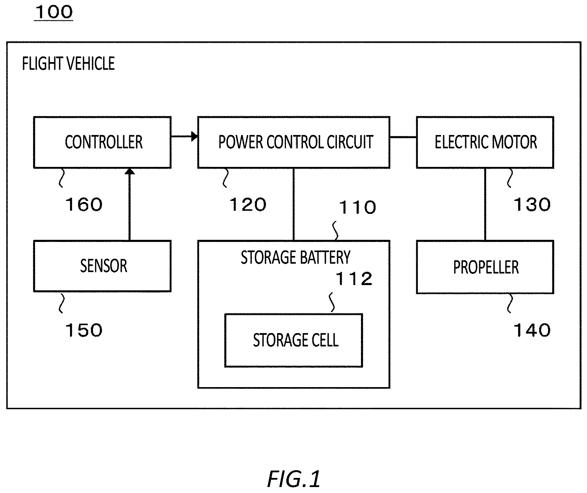

FIG. 1 schematically illustrates an example of a system configuration of the flight vehicle 100. In this embodiment, the flight vehicle 100 includes a storage battery 110, the power control circuit 120, one or more electric motors 130, one or more propellers 140, one or more sensors 150, and a controller 160. In this embodiment, the storage battery 110 has one or more storage cells 112.

In this embodiment, the flight vehicle 100 flies using electrical energy stored in the storage battery 110. Examples of the flight vehicle 100 include an airplane, an airship or a balloon, an air balloon, the helicopter, a drone, or the like.

In this embodiment, the storage battery 110 receives electrical energy from the external charging device (not illustrated) via the power control circuit 120, and stores the electrical energy in one or more storage cells 112. In addition, the storage battery 110 supplies, via the power control circuit 120, the electrical energy stored in one or more storage cells 112 to the electric motor 130.

In this embodiment, the storage cell 112 stores electrical energy (which may be referred to as charging of the storage cell 112). The storage cell 112 also releases the stored electrical energy (which may be referred to as discharging of the storage cell 112). The storage cell 112 may be the secondary battery. The storage cell 112 may be the nonaqueous secondary battery.

Examples of the nonaqueous secondary battery include a sodium-ion secondary battery, a lithium-ion secondary battery, a lithium metal secondary battery, a lithium-air secondary battery, a lithium-sulfur secondary battery, a magnesium-ion secondary battery, an aluminum-ion secondary battery, and the like. The lithium-ion secondary battery, which is one aspect of the nonaqueous secondary battery, may be a concept including the nonaqueous lithium-ion secondary battery using a nonaqueous electrolytic solution and an all-solid-state lithium-ion secondary battery using a solid electrolyte.

For example, as the secondary battery active material mounted on a vehicle, a material having a large amount of electric charge that can be stored per unit volume is often selected. In contrast, in this embodiment, the storage cell 112 is mounted on the flight vehicle 100. Therefore, the active material used for the storage cell 112 is preferably the material having a large amount of electric charge that can be stored per unit mass.

The gravimetric energy density of the storage cell 112 is preferably 500 [Wh/kg- storage cell] or more, more preferably 550 [Wh/kg-storage cell] or more, more preferably 600 [Wh/kg-storage cell] or more, more preferably 650 [Wh/kg- storage cell] or more, and further preferably 700 [Wh/g-storage cell] or more. In this way, the storage cell particularly suitable for the application of the power supply for the flight vehicle is obtained.

The volume energy density of the storage cell 112 may be 300 [Wh/m³-storage cell] or more and 1200 [Wh/m³-storage cell] or less or may be 400 [Wh/m³-storage cell] or more and 1000 [Wh/m³-storage cell] or less. If the storage cell 112 is mounted on the flight vehicle 100 as a part of the power supply of the flight vehicle 100, the volume energy density of the storage cell 112 may be 600 [Wh/m³-storage cell] or less or may be 800 [Wh/m³-storage cell] or less.

The storage cell 112 may have a gravimetric energy density within the above-described numerical range and a volume energy density within the above-described numerical range. In this way, the storage cell that is relatively difficult to use for the power supply of the vehicle can be used as the power supply of the flight vehicle. The details of the storage cell 112 will be described below.

In this embodiment, the power control circuit 120 controls the input and output of the electrical power of the storage battery 110. The power control circuit 120 may control the input and output of the electrical power of the storage battery 110 based on the instruction from the controller 160. For example, the power control circuit 120 includes a plurality of switching devices that operate based on the control signal from the controller 160.

In this embodiment, the electric motor 130 receives the electrical energy from the storage battery 110 via the power control circuit 120. The electric motor 130 uses the electrical energy received from the storage battery 110 to rotate the propeller 140. In this way, the electric motor 130 can generate the propulsive force of the flight vehicle 100 using the electrical energy accumulated in the storage cell 112.

In this embodiment, the sensor 150 measures various physical quantities related to the position and posture of the flight vehicle 100. Examples of sensors for measuring the various physical quantities related to the position and posture of the flight vehicle 100 include a GPS signal receiver, an acceleration sensor, an angular acceleration sensor, a gyro sensor, or the like. The sensor 150 may measure various physical quantities related to the state of the storage battery 110. Examples of a sensor for measuring various physical quantities related to the state of the storage battery 110 include a temperature sensor, a current sensor, a voltage sensor, or the like.

In this embodiment, the controller 160 controls the flight vehicle 100. The controller 160 may control the input and output of the electrical power of the storage battery 110 by controlling the power control circuit 120. For example, the controller 160 controls the output current, the output voltage, the input current, the input voltage, or the like of the storage battery 110. In this way, the controller 160 can control the position and posture of the flight vehicle 100. The controller 160 may control the position and posture of the flight vehicle 100 by controlling the power control circuit 120 based on the output from the sensor 150.

The storage battery 110 may be one example of a secondary battery. The storage cell 112 may be one example of a secondary battery. The electric motor 130 may be one example of a propulsive force generator.

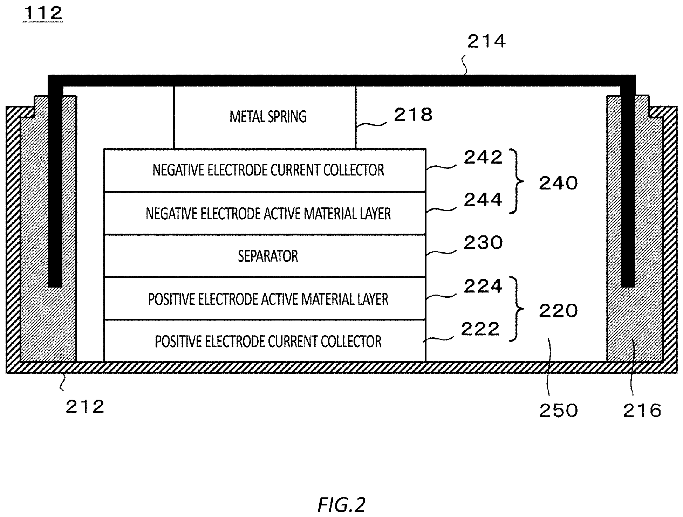

Fig. 2 schematically illustrates one example of the storage cell 112. In this embodiment, details of the storage cell 112 will be described by taking, as an example, a case where the storage cell 112 is the coin-type nonaqueous secondary battery.

### (Power Storage Cell)

In this embodiment, the storage cell 112 includes the positive electrode case 212, the negative electrode case 214, the sealant 216, and the metal spring 218. The storage cell 112 also includes the positive electrode 220, the separator 230, the negative electrode 240, and the electrolytic solution 250. In this embodiment, the positive electrode 220 has the positive electrode current collector 222 and the positive electrode active material layer 224. In this embodiment, the negative electrode 240 has the negative electrode current collector 242 and the negative electrode active material layer 244.

In this embodiment, by assembling the positive electrode case 212 and the negative electrode case 214, the space is formed inside the positive electrode case 212 and the negative electrode case 214. Inside the space formed by the positive electrode case 212 and the negative electrode case 214, the metal spring 218, the positive electrode 220, the separator 230, the negative electrode 240, and the electrolytic solution 250 are accommodated. The positive electrode 220, the separator 230, and the negative electrode 240 are fixed inside the positive electrode case 212 and the negative electrode case 214 by the repulsive force of the metal spring 218.

The positive electrode case 212 and the negative electrode case 214 are made of, for example, the conductive material having a disk-shaped thin plate shape. In this embodiment, the sealant 216 seals the gap formed between the positive electrode case 212 and the negative electrode case 214. The sealant 216 includes an insulating material. The sealant 216 insulates the positive electrode case 212 and the negative electrode case 214.

### (Positive Electrode)

In this embodiment, the positive electrode current collector 222 holds the positive electrode active material layer 224. Examples of the material of the positive electrode current collector 222 include aluminum, stainless steel, nickel, titanium, or alloys thereof or the like. Examples of the shape of the positive electrode current collector 222 include foil, mesh, punched metal, expanded metal, and the like. The thickness of the positive electrode current collector 222 is not particularly limited, but is preferably 5 to 200 µm. The thickness of the positive electrode current collector 222 may be 6 to 20 µm.

In this embodiment, the positive electrode active material layer 224 is formed on at least one surface of the positive electrode current collector 222. The thickness of the positive electrode active material layer 224 may be 1 to 300 µm per one surface of the positive electrode current collector 222, and may be 2 to 200 µm. The positive electrode active material layer 224 includes, for example, the positive electrode active material and a binding agent(which may be referred to as a binder). The positive electrode active material layer 224 may include a conductive additive.

In one embodiment, the positive electrode active material layer 224 is formed by applying, onto at least one surface of the positive electrode current collector 222, a paste containing a material constituting the positive electrode active material layer 224 and the organic solvent, and drying the paste. The type of the organic solvent described above is not particularly limited, but examples of the organic solvent described above include N-methylpyrrolidone (NMP) and the like. In another embodiment, the positive electrode active material layer 224 is formed by mixing materials constituting the positive electrode active material layer 224, molding them into a sheet shape, and pressing the sheet-shaped mixture onto at least one surface of the positive electrode current collector 222.

The positive electrode active material layer 224 may contain, as the positive electrode active material, a heterocyclic compound containing one or more pyrazine rings and two or more benzene rings, or a salt or derivative thereof. The heterocyclic compound described above may be the compound containing one or more phenazine structures. The phenazine structure has a structure that (i) includes one pyrazine ring and two benzene rings, and (ii) each of the two benzene rings shares one carbon-carbon bond of the pyrazine ring with the pyrazine ring and is linked to the pyrazine ring. The pyrazine ring described above may be substituted with any functional group, or may be unsubstituted. The benzene ring described above may be substituted with any functional group, or may be unsubstituted.

The heterocyclic compound described above preferably includes a structure in which at least four oxygen atoms are bonded to the benzene ring described above. Thus, the heterocyclic compound described above can accumulate more charge even with a small molecular weight. As a result, by using the heterocyclic compound described above as the positive electrode active material, the storage cell having a large energy density per unit mass of the storage cell can be obtained.

The heterocyclic compound described above is preferably the structure in which an even number of oxygen atoms of 4 or more are bonded to the benzene ring described above.

The positive electrode active material layer 224 may contain phenazines as the positive electrode active material. The phenazines described above are preferably compounds in which at least four oxygen atoms are bonded to the benzene ring included in a phenazine structure. As described above, this makes it possible to obtain the storage cell having a high energy density per unit mass of the storage cell.

The phenazines described above are preferably compounds in which an even number of oxygen atoms of four or more are bonded to the benzene ring described above. The phenazines described above may be compounds in which an even number of oxygen atoms of four or more and eight or less are bonded to the benzene ring described above. This improves the chemical stability of the heterocyclic compound.

The positive electrode active material layer 224 may include, as the positive electrode active material, an oligomer in which a plurality of phenazine structures are bonded. The oligomer may be the oligomer in which a plurality of phenazine structures are bonded via a linker. This suppresses dissolution of the positive electrode active material into the electrolytic solution during charging and discharging, and improves cycle performance of the secondary battery.

The positive electrode active material described above is made of, for example, the heterocyclic compound including one or more pyrazine rings and two or more benzene rings, or a salt or derivative thereof. The positive electrode active material described above contains, as a main component, for example, the heterocyclic compound including one or more pyrazine rings and two or more benzene rings, or a salt or derivative thereof.

The above heterocyclic compound or a salt or derivative thereof, phenazines, and oligomers thereof are included, for example, in any of a reaction starting material, a product, and an intermediate product in at least the discharge reaction of the battery electrode reaction. Details of the phenazine oligomers that can be used as a positive electrode active material, or salts or derivatives thereof, will be described later.

The positive electrode active material is not limited to the above heterocyclic compound and the like. When the storage cell 112 is a lithium-ion secondary battery or a lithium metal secondary battery, other examples of the positive electrode active material include layered oxides such as LiMnO₂, LiNiO₂, LiCoO₂, Li(MnₓNi₁₋ₓ)O₂, Li(MnₓCo₁₋ₓ)O₂, Li(Ni_{y}Co_{1-y})O₂, and Li(MnₓNi_{y}Co_{1-x-y})O₂; solid solutions such as Li₂MnO₃-LiNiO₂, Li₂MnO₃-LiCoO₂, and Li₂MnO₃-Li(Ni_{y}Co_{1-y})O₂; silicates such as Li₂MnSiO₄, Li₂NiSiO₄, Li₂CoSiO₄, Li₂(MnₓNi₁₋ₓ)SiO₄, Li₂(MnₓCo₁₋ₓ)SiO₄, Li₂(Ni_{y}Co_{1-y})SiO₄, and Li₂(MnₓNi_{y}Co_{1-x-y})SiO₄; borates such as LiMnBO₃, LiNiBO₃, LiCoBO₃, Li(MnₓNi₁₋ₓ)BO₃, Li(MnₓCo₁₋ₓ)BO₃, Li(Ni_{y}Co_{1-y})BO₃, and Li(MnₓNi_{y}Co_{1-x-y})BO₃; V₂O₅; LiV₃O₆; MnO; and the like. In the above formula, 0<x<1, 0<y<1, and 0<x+y<1. These positive electrode active materials may be used alone, or two or more types of positive electrode active materials may be combined.

When the storage cell 112 is a sodium-ion secondary battery, other examples of the positive electrode active material include NaFeO₂, NaNiO₂, NaCoO₂, NaMnO₂, NaVO₂, Na(Ni_{X}Mn_{1-X})O₂, Na(Fe_{X}Mn_{1-X})O₂, NaVPO₄F, Na₂FePO₄F, Na₃V₂(PO₄)₃, and the like. In the above formula, 0<x<1. These positive electrode active materials may be used alone, or two or more types of positive electrode active materials may be combined.

In this embodiment, the binding agent binds materials (for example, the positive electrode active material, the conductive additive, and the like) constituting the positive electrode active material layer 224, and maintains the electrode shape of the positive electrode 220. The type of the binding agent is not particularly limited, but examples of the binding agent include polyvinylidene fluoride (PVDF), polytetrafluoroethylene, polyacrylic acid, styrene butadiene rubber, and the like.

In this embodiment, the conductive additive reduces resistance of the positive electrode 220. The type of the conductive additive is not particularly limited as long as it has desired electronic conductivity, and carbon materials are exemplified as the conductive additive. Examples of the carbon materials include graphite, carbon black (e.g., acetylene black, Ketjen black, and the like), coke, amorphous carbon, carbon fibers, carbon nanotubes, graphene, and the like. These conductive additives may be used alone, or two or more types of conductive additives may be combined.

When the positive electrode active material layer 224 does not contain the conductive additive, the content of the positive electrode active material in the positive electrode active material layer 224 is preferably 40 to 99 mass%, more preferably 70 to 97 mass%, and further preferably 80 to 95 mass%. The content of the binding agent in the positive electrode active material layer 224 is preferably 1 to 60 mass%, more preferably 3 to 30 mass%, and further preferably 5 to 20 mass%.

When the positive electrode active material layer 224 contains a conductive additive, the content of the conductive additive in the positive electrode active material layer 224 is preferably 0.2 to 20 mass%, more preferably 1 to 10 mass%, and further preferably 2 to 6 mass%. At this time, the content of the positive electrode active material in the positive electrode active material layer 224 is preferably 60 to 98 mass%, more preferably 70 to 96 mass%, and further preferably 80 to 94 mass%. Further, the content of the binding agent in the positive electrode active material layer 224 is preferably 1.8 to 39.8 mass%, more preferably 3 to 29 mass%, and further preferably 4 to 18 mass%.

Note that the content of the binding agent in the positive electrode active material layer 224 may be 1 to 20 mass%, may be 2 to 10 mass%, or may be 3 to 6 mass%. In this case, the remainder of the positive electrode active material layer 224 may be the positive electrode active material, or may be a mixture of the positive electrode active material and the conductive additive.

### (Separator)

In this embodiment, the separator 230 separates the positive electrode 220 and the negative electrode 240. The separator 230, for example, ensures ion conductivity between the positive electrode 220 and the negative electrode 240 by holding the electrolytic solution. Examples of the material of the separator 230 include polyethylene, polypropylene, the ethylene-propylene copolymer, glass, or composites thereof. Examples of the shape of the separator 230 include the microporous film, the nonwoven fabric, the filter, and the like. The thickness of the separator 230 is not particularly limited, but is preferably 10 to 50 µm. The porosity of the separator 230 is not particularly limited, but is preferably 30 to 70%.

### (Negative Electrode)

In this embodiment, the negative electrode current collector 242 holds the negative electrode active material layer 244. Examples of the material of the negative electrode current collector 242 include copper, aluminum, stainless steel, nickel, titanium, or alloys thereof or the like. The negative electrode current collector 242 may include a resin support layer and a metal layer disposed on the surface of the support layer. Examples of the resin described above include polyethylene, polypropylene, polyethylene terephthalate, polyimide, and the like. The metal layer described above may be the layer made of copper, aluminum, stainless steel, nickel, titanium, or an alloy thereof. The metal layer described above may include the layer made of copper, aluminum, stainless steel, nickel, titanium, or an alloy thereof. The metal layer may be the foil or may be the plating layer.

When lithium metal is used as the negative electrode active material, the lithium metal can also serve as the current collector. Therefore, when the storage cell 112 is a lithium metal secondary battery, the storage cell 112 may not include the negative electrode current collector 242.

Examples of the shape of the negative electrode current collector 242 include foil, mesh, punched metal, expanded metal, and the like. The thickness of the negative electrode current collector 242 is not particularly limited, but may be 5 to 200 µm. The thickness of the negative electrode current collector 242 is preferably 6 to 20 µm.

In this embodiment, the negative electrode active material layer 244 is formed on at least one surface of the negative electrode current collector 242. The thickness of the negative electrode active material layer 244 may be 1 to 300 µm per one surface of the negative electrode current collector 242, and may be 2 to 200 µm. The negative electrode active material layer 244 includes, for example, the negative electrode active material and the binding agent. The negative electrode active material layer 244 may include the conductive additive.

In one embodiment, the negative electrode active material layer 244 is formed by applying, onto at least one surface of the negative electrode current collector 242, a paste including a material constituting the negative electrode active material layer 244 and the organic solvent, and drying the paste. The type of the organic solvent described above is not particularly limited, but examples of the organic solvent described above include N-methylpyrrolidone (NMP) and the like. In another embodiment, the negative electrode active material layer 244 is formed by mixing materials constituting the negative electrode active material layer 244 to mold the mixture into a sheet shape, and pressing the sheet-shaped mixture to at least one surface of the negative electrode current collector 242.

The negative electrode active material layer 244 may include, as a negative electrode active material, the heterocyclic compound including one or more pyrazine rings and two or more benzene rings, or a salt or derivative thereof. The heterocyclic compound described above may be the compound including one or more phenazine structures. The phenazine structure (i) includes one pyrazine ring and two benzene rings, and (ii) has a structure in which each of the two benzene rings shares one carbon-carbon bond of the pyrazine ring with the pyrazine ring and is linked to the pyrazine ring. The pyrazine ring described above may be substituted with any functional group, or may be unsubstituted. The benzene ring described above may be substituted with any functional group, or may be unsubstituted.

Similarly to the heterocyclic compound described in relation to the positive electrode active material layer 224, the heterocyclic compound that can be used in the negative electrode active material layer 244 is preferably the compound in which at least four oxygen atoms are bonded to the benzene ring described above. The heterocyclic compound described above is preferably the compound in which an even number of oxygen atoms of 4 or more are bonded to the benzene ring described above. The heterocyclic compound described above may be the compound in which an even number of oxygen atoms of 4 or more are bonded to the benzene ring described above.

The negative electrode active material layer 244 may contain phenazines as the negative electrode active material. The phenazines described above are preferably compounds in which at least four oxygen atoms are bonded to the benzene ring included in a phenazine structure. The phenazine structure described above is preferably the compound in which an even number of oxygen atoms of 4 or more are bonded to the benzene ring described above. The phenazine structure described above may be the compound in which an even number of oxygen atoms of 4 or more and 8 or less are bonded to the benzene ring described above.

The negative electrode active material described above may include the oligomer in which a plurality of phenazine structures are bonded. The oligomer may be the oligomer in which a plurality of phenazine structures are bonded via a linker. This suppresses dissolution of the positive electrode active material into the electrolytic solution in the secondary battery, and improves cycle performance of the secondary battery.

The above heterocyclic compound or a salt or derivative thereof, and phenazines and oligomers thereof are contained, for example, in any of a reaction starting material, a product, and an intermediate product in at least the charge reaction of the battery electrode reaction. Details of the oligomer of phenazines that can be used as a negative electrode active material, or a salt or derivative thereof, will be described later.

The negative electrode active material is not limited to the above heterocyclic compound and the like. When the storage cell 112 is a lithium-ion secondary battery, other examples of the negative electrode active material include (i) graphite, (ii) non-sinterable carbon or non-graphitizable carbon, (iii) tin, silicon, and alloys containing these, and (iv) SiO and the like. When materials such as (i) graphite, (ii) non-sinterable carbon or non-graphitizable carbon, (iii) tin, silicon, and alloys containing these, and (iv) SiO and the like are used as the negative electrode active material, the materials may be pre-doped with lithium.

The negative electrode active material may be the lithium-containing material such as metallic lithium or a lithium alloy. For example, when the storage cell 112 is a lithium metal secondary battery, metallic lithium is used as the negative electrode. These negative electrode active materials may be used alone, or two or more types of negative electrode active materials may be combined.

The negative electrode active material layer 244 may include the lithium metal foil. Thus, lithium is supplied to the storage cell 112. The thickness of the lithium metal foil may be 1 to 300 µm, may be 2 to 200 µm, and may be 3 to 100 µm. The thickness and/or mass of the lithium metal foil may be determined according to the content of the positive electrode active material in the positive electrode active material layer 224.

When the storage cell 112 is a sodium-ion secondary battery, other examples of the negative electrode active material include (i) graphite, (ii) non-sinterable carbon or non-graphitizable carbon, (iii) tin, silicon, and alloys containing these, (iv) titanium oxides, and the like. The negative electrode active material may be the sodium-containing material such as metallic sodium or a sodium alloy. These negative electrode active materials may be used alone, or two or more negative electrode active materials may be combined.

In this embodiment, the binding agent binds materials constituting the negative electrode active material layer 244 (for example, the negative electrode active material, the conductive additive, and the like) and maintains the electrode shape of the negative electrode 240. The type of the binding agent is not particularly limited; however, examples of the binding agent include polyvinylidene fluoride (PVDF), polytetrafluoroethylene, polyacrylic acid, styrene butadiene rubber, and the like.

In this embodiment, the conductive additive reduces the resistance of the negative electrode 240. The type of the conductive additive is not particularly limited as long as it has desired electronic conductivity, and carbon materials are exemplified as the conductive additive. Examples of the carbon material include graphite, carbon black (for example, acetylene black, Ketjen black, and the like), coke, amorphous carbon, carbon fibers, carbon nanotubes, graphene, and the like. These conductive additives may be used alone, or two or more conductive additives may be combined.

When the negative electrode active material layer 244 does not contain the conductive additive, the content of the negative electrode active material in the negative electrode active material layer 244 is preferably 40 to 99 mass%, more preferably 80 to 98.5 mass%, and still more preferably 90 to 98 mass%. The content of the binding agent in the negative electrode active material layer 244 is preferably 1 to 60 mass%, more preferably 1.5 to 20 mass%, and still more preferably 2 to 10 mass%.

When the negative electrode active material layer 244 contains a conductive additive, the content of the conductive additive in the negative electrode active material layer 244 is preferably 0.1 to 20 mass%, more preferably 1 to 10 mass%, and still more preferably 2 to 5 mass%. At this time, the content of the negative electrode active material in the negative electrode active material layer 244 is preferably 40 to 98 mass%, more preferably 80 to 97 mass%, and still more preferably 90 to 96 mass%. Further, the content of the binding agent in the negative electrode active material layer 244 is preferably 1.9 to 59.9 mass%, more preferably 2 to 19 mass%, and still more preferably 2 to 8 mass%.

The content of the binding agent in the negative electrode active material layer 244 may be 0.1 to 5 mass%, may be 0.2 to 3 mass%, or may be 0.5 to 1 mass%. In this case, the remainder of the negative electrode active material layer 244 may be the negative electrode active material, or may be a mixture of the negative electrode active material and the conductive additive.

### (Electrolyte)

In this embodiment, the electrolytic solution 250 achieves ion conduction between the positive electrode active material and the negative electrode active material via the electrolyte contained in the electrolytic solution 250. According to this embodiment, the nonaqueous electrolytic solution is used as the electrolytic solution 250. As the nonaqueous electrolytic solution, the known organic electrolytic solution can be used. For example, when the storage cell 112 is a lithium-ion secondary battery or a lithium metal secondary battery, a solution in which (ii) a lithium salt such as lithium perchlorate, LiPF₆, or the like is dissolved in (i) the solvent composed of one or more of ethylene carbonate, dimethyl carbonate, diethyl carbonate, and the like is used as the electrolytic solution 250.

The nonaqueous electrolytic solution contains, for example, the metal salt and the nonaqueous solvent. Examples of the metal salt include the sodium salt, the lithium salt, and the like. Examples of the sodium salt include inorganic sodium salts such as NaPF₆, NaBF₄, NaClO₄, and NaAsF₆, and organic sodium salts such as NaCF₃SO₃, NaN(CF₃SO₂)₂, NaN(C₂F₅SO₂)₂, and NaC(CF₃SO₂)₃. Examples of the lithium salt include inorganic lithium salts such as LiPF₆, LiBF₄, LiClO₄, and LiAsF₆, and organic lithium salts such as LiCF₃SO₃, LiN(CF₃SO₂)₂, LiN(C₂F₅SO₂)₂, and LiC(CF₃SO₂)₃. Examples of the nonaqueous solvent include ethylene carbonate (EC), propylene carbonate (PC), dimethyl carbonate (DMC), diethyl carbonate (DEC), ethyl methyl carbonate (EMC), butylene carbonate (BC), fluoroethylene carbonate (FEC), γ-butyrolactone, sulfolane, acetonitrile, 1,2-dimethoxymethane, 1,3-dimethoxypropane, diethyl ether, tetrahydrofuran, 2-methyltetrahydrofuran, and mixtures thereof.

The concentration of the sodium salt in the nonaqueous electrolytic solution is not particularly limited, but may be within a range of 0.5 to 4.0 mol/L. The concentration of the sodium salt may be within the range of 0.7 mol/L to 2.0 mol/L, and may be within the range of 1.0 mol/L to 1.5 mol/L. The concentration of the lithium salt in the nonaqueous electrolytic solution is not particularly limited, but may be within the range of 0.5 to 4.0 mol/L. The concentration of the lithium salt may be within the range of 0.7 mol/L to 2.0 mol/L, and may be within the range of 1.0 mol/L to 1.5 mol/L.

### (An example of another embodiment)

In this embodiment, details of the storage cell 112 have been described by taking as an example a case where the storage cell 112 is the coin-type secondary battery. However, the type, structure, and the like of the storage cell 112 are not limited to this embodiment. In another embodiment, the storage cell 112 may be a cylindrical battery including a wound electrode body in which the positive electrode, the separator, and the negative electrode are spirally wound. In still another embodiment, the storage cell 112 may be the laminate-type battery in which the stacked electrode body in which positive electrodes and negative electrodes are alternately stacked with separators interposed therebetween is sealed with a laminate.

In this embodiment, details of the storage cell 112 have been described by taking as an example a case where the negative electrode 240 includes the negative electrode current collector 242 and the negative electrode active material layer 244. However, the negative electrode of the storage cell 112 is not limited to this embodiment. In another embodiment, for example, when the storage cell 112 is a lithium metal secondary battery, metallic lithium can be used as the negative electrode.

In this embodiment, an example of the storage cell 112 has been described by taking as an example a case where the electrolytic solution 250 is used as an electrolyte of the storage cell 112. However, the electrolyte of the storage cell 112 is not limited to this embodiment. In another embodiment, the solid electrolyte or a gel electrolyte may be used as the electrolyte of the storage cell 112. Examples of the solid electrolyte include inorganic solid electrolytes such as the Li₂S-P₂S₅-based system and the Li₂S-GeS₂-P₂S₅-based system.

As described above, details of the flight vehicle 100 and the storage cell 112 have been described with reference to FIGS. 1 and 2. Next, details of the secondary battery active material used as a positive electrode active material or a negative electrode active material will be described.

### (I. Active Material)

As described above, according to this embodiment, the oligomer in which a plurality of phenazine structures are bonded is used as a positive electrode active material or a negative electrode active material for a secondary battery (which may be simply referred to as the active material).

In this embodiment, the above-described active material includes at least one compound represented by the following General Formula (1), General Formula (2), General Formula (3), and General Formula (4), or a salt thereof. The above-described compound or a salt thereof may be included in any of a reaction starting material, a product, and an intermediate product in the discharge reaction of the battery electrode reaction. The above-described compound or a salt thereof may be included in any of the reaction starting material, the product, and the intermediate product in the charge reaction of the battery electrode reaction.

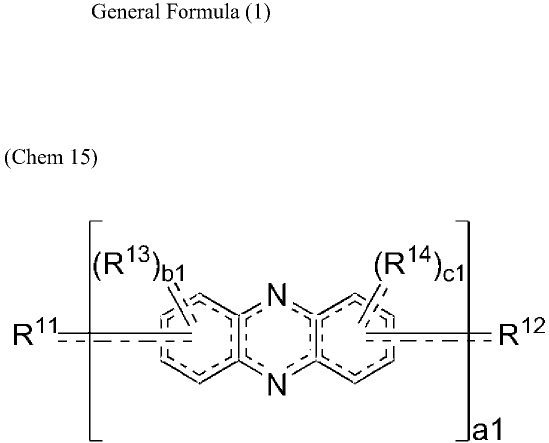

In General Formula (1), R¹¹ at a terminal portion, R¹² at a terminal portion, R¹³, and R¹⁴ may each independently indicate an oxygen atom, a group represented by -OM (which may be referred to as the OM group), a hydrogen atom, or an organic group. M may indicate a hydrogen atom, or the monovalent or divalent metal atom. In the OM group, the bond between O and the metal atom may be an ionic bond.

When M is the divalent metal atom, two adjacent ones of R¹¹ to R¹⁴ may be linked to each other to form a ring represented by -OMO-. At this time, R¹¹ at a terminal portion may be linked to R¹³ bonded to the benzene ring to which R¹¹ is bonded, and R¹² at a terminal portion may be linked to R¹⁴ bonded to the benzene ring to which R¹² is bonded. For example, when M is Mg, R¹¹ and R¹³ adjacent thereto may form the ring represented by -OMgO-.

A double line indicated by a solid line and a dashed line indicates the single bond or the double bond. In General Formula (1), the single bond and the double bond may be arranged such that the compound is the conjugated molecule and has a resonance structure.

In this embodiment, square brackets in a chemical formula represent a repeating unit in a molecule. A solid line and a dashed line passing through the square brackets indicate a bond between repeating units or a bond between the repeating unit and a terminal group (in the case of General Formula (1), R¹¹ and R¹²).

In General Formula (1), a1 indicates the number of repeating units of the phenazine structure. a1 may be the integer from 2 to 10. When a1 is within this range, it is possible to suppress dissolution of the active material into the electrolytic solution during charging and discharging and to improve cycle performance of the secondary battery. In addition, it is possible to suppress a decrease in crystallinity of the active material and a decrease in electronic conductivity.

In General Formula (1), b1 and c1 may each independently be the integer from 1 to 3. When b1 and c1 are 2 or 3, two or three R¹³ and R¹⁴ may each be the same as to or different from each other, and at least four of R¹³ and R¹⁴ may be an oxygen atom or a group represented by -OM. The number of oxygen atoms or OM groups among R¹³ and R¹⁴ may be the even number of 4 or more. All of R¹³ and R¹⁴ may be oxygen atoms or OM groups.

This increases the number of electrons involved in the redox reaction. Specifically, the multi-electron reaction of 4 or more electrons becomes possible in the redox reaction, and the theoretical capacity of the active material increases. Therefore, the active material can accumulate more charge even with the small molecular weight. As a result, the storage cell having a large energy density per unit mass of the storage cell is obtained.

In a state in which the active material is fully charged, the bond between an oxygen atom included in R¹¹ to R¹⁴ described above and a carbon of a benzene ring included in the phenazine structure is a double bond. At this time, the atom represented by M described above is not bonded to a nitrogen atom included in a pyrazine ring of the phenazine structure. For example, a hydrogen ion or a metal ion is not bonded to the nitrogen atom described above.

On the other hand, in a state in which the active material is fully discharged, all oxygen atoms included in R¹¹ to R¹⁴ described above are in a state of being present as OM groups, and the bond between an oxygen atom of the OM group and a carbon of a benzene ring included in the phenazine structure is a single bond. In this case, the atom represented by M described above is bonded to the nitrogen atom contained in the pyrazine ring of the phenazine structure. For example, the hydrogen ion or the metal ion is bonded to the nitrogen atom described above.

The organic groups indicated by R¹¹ to R¹⁴ may be substituted or unsubstituted hydrocarbon groups. The hydrocarbon group described above may be the monovalent hydrocarbon group. In the organic groups indicated by R¹¹ to R¹⁴, two adjacent organic groups may be linked to each other to form a ring.

Examples of the organic group described above include the substituted or unsubstituted alkyl group, the substituted or unsubstituted alkenyl group, the substituted or unsubstituted alkynyl group, the substituted or unsubstituted alkoxy group, the substituted or unsubstituted carboxy group, the substituted or unsubstituted alkoxycarbonyl group, the substituted or unsubstituted acyl group, the substituted or unsubstituted acyloxy group, the substituted or unsubstituted aryl group, the substituted or unsubstituted aryloxy group, the substituted or unsubstituted heteroaryl group, the substituted or unsubstituted heteroaryloxy group, the substituted or unsubstituted ester group, the substituted or unsubstituted ether group, the substituted or unsubstituted amino group, the substituted or unsubstituted sulfonic acid group, the substituted or unsubstituted cyano group, the substituted or unsubstituted thioether group, and the like. The organic group described above may be the monovalent group having an ester bond (-COO-), and may be the monovalent group having an ether bond (-O-). The organic group described above may contain boron. The organic group described above may contain boron as the hetero atom.

The organic group described above may be constituted by one or more atoms selected from the group consisting of carbon, hydrogen, oxygen, nitrogen, and boron. These atoms have smaller atomic weights than, for example, sulfur. Therefore, when the above-described organic group is composed of carbon, hydrogen, oxygen, nitrogen, and boron, the molecular weight of the compound used as the active material becomes relatively small. As a result, the gravimetric energy density of the active material or the storage cell is improved. The organic group described above may be composed of one or more atoms selected from the group consisting of carbon, hydrogen, oxygen, and nitrogen.

When the number of electrons involved in the redox reaction in the active material is the same, an increase in the molecular weight of the active material decreases the amount of electric charge that can be stored per unit mass. Therefore, when the above-described organic group is the substituted or unsubstituted hydrocarbon group, the number of carbon atoms of the organic group is preferably 1 to 6, more preferably less than 4, and still more preferably 2 or less. The number of carbon atoms of the organic group described above may be 1.

Examples of the above-described alkyl group include alkyl groups having 1 to 6 carbon atoms, such as the methyl group, the ethyl group, the n-propyl group, the isopropyl group, the n-butyl group, the isobutyl group, the sec-butyl group, and the tert-butyl group. The number of carbon atoms of the alkyl group is particularly preferably 1 to 3. The above-described alkyl group may be the linear alkyl group or may be a branched-chain alkyl group.

Examples of the alkoxy group described above include alkoxy groups having 1 to 6 carbon atoms, such as the methoxy group, the ethoxy group, the n-propoxy group, the isopropoxy group, the n-butyloxy group, the isobutyloxy group, the sec-butyloxy group, and the tert-butyloxy group. The number of carbon atoms of the alkoxy group is particularly preferably 1 to 3. The alkoxy group described above may be the linear alkoxy group or may be a branched alkoxy group.

Examples of the aryl group described above include the phenyl group, the naphthyl group, the anthranyl group, the phenanthryl group, the biphenyl group, and the pyridyl group. The aryl group is particularly preferably the phenyl group.

In the OM group indicated by R¹¹ to R¹⁴, M may be H, Li, Na, K, Mg, or Ca. The type of the metal atom indicated as M may be the same as the type of the metal atom constituting the metal ion that moves between the positive electrode and the negative electrode of the secondary battery. For example, when the secondary battery is a lithium-ion secondary battery or a lithium metal secondary battery, M indicates a hydrogen atom or a lithium atom. When the secondary battery is a sodium-ion battery, M indicates a hydrogen atom or a sodium atom.

As described above, when the number of electrons involved in an redox reaction in the active material is the same, an increase in the molecular weight of the active material reduces the amount of electric charge that can be stored per unit mass. Therefore, as the ratio of the molecular weight of the compound to the number of electrons involved in the redox reaction in the compound represented by General Formula (1) is smaller, the volume energy density of the compound can be larger. In consideration of ease of synthesis and the amount of electric charge that can be stored per unit mass, those other than an oxygen atom or an OM group among R¹¹ to R¹⁴ are preferably hydrogen atoms.

The above General Formula (1) may be represented, for example, by the following chemical formula (1A). Chemical Formula (1A)

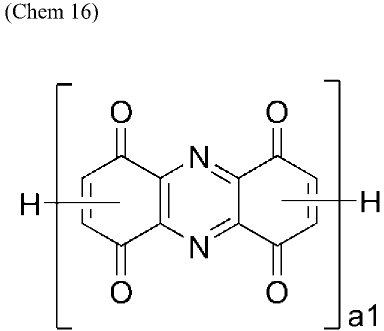

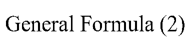

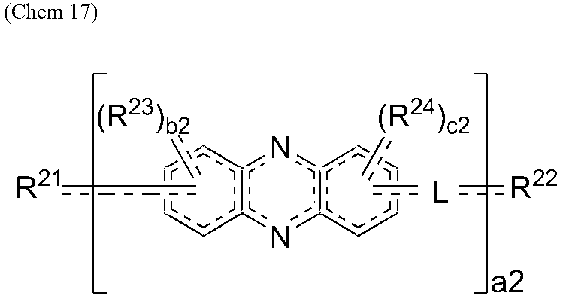

In General Formula (2), terminal R²¹, terminal R²², R²³, and R²⁴ may each independently represent the oxygen atom, the group represented by -OM, the hydrogen atom, or the organic group. M may represent a hydrogen atom, or the monovalent or divalent metal atom. When M is the divalent metal atom, two adjacent ones of R²¹ to R²⁴ may be linked to each other to form a ring represented by -OMO-. The ring represented by -OMO- may have the same configuration as -OMO- described above for General Formula (1).

L is a direct bond or the divalent linker that includes an aromatic group, a double bond, or a triple bond. The linker may be an organic group including an arylene group, a heterocyclic group, an alkenylene group, or an alkynylene group. The linker may be, for example, the phenylene group, an ethene-1,2-diyl group, or an acetylene-1,2-diyl group. In General Formula (2), L may be the same as or different from each other for each repeating unit described in brackets.

A double line indicated by a solid line and a dashed line indicates the single bond or the double bond. The single bond and the double bond may be arranged such that the compound is the conjugated molecule and has the resonance structure.

In General Formula (2), a2 indicates the number of repeating units each including a phenazine structure and the linker included in the oligomer. a2 may be the integer from 2 to 10.

In General Formula (2), b2 and c2 may each independently be an integer from 1 to 3. When b2 and c2 are 2 or 3, two or three R²³ and R²⁴ may each be the same as or different from each other, and at least four of R²³ and R²⁴ may be an oxygen atom or a group represented by -OM. The number of oxygen atoms or OM groups among R²³ and R²⁴ may be the even number of 4 or more. All of R²³ and R²⁴ may be oxygen atoms or OM groups.

Each of R²¹ to R²⁴ may have the same configuration as R¹¹ to R¹⁴ described above. For example, R²¹ to R²⁴ each independently represent an oxygen atom, a group represented by -OM, a hydrogen atom, or an organic group. Further, each of R²¹ to R²⁴ may be determined such that the compound represented by General Formula (2) has symmetry. Each of R²¹ to R²⁴ may be determined such that the compound represented by General Formula (2) has the axis of symmetry, the plane of symmetry, or the center of symmetry.

The above General Formula (2) may be represented, for example, by the following chemical formula (2A). Chemical Formula (2A)

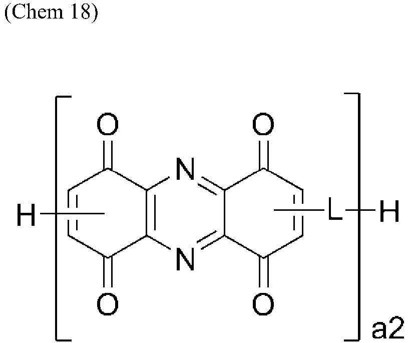

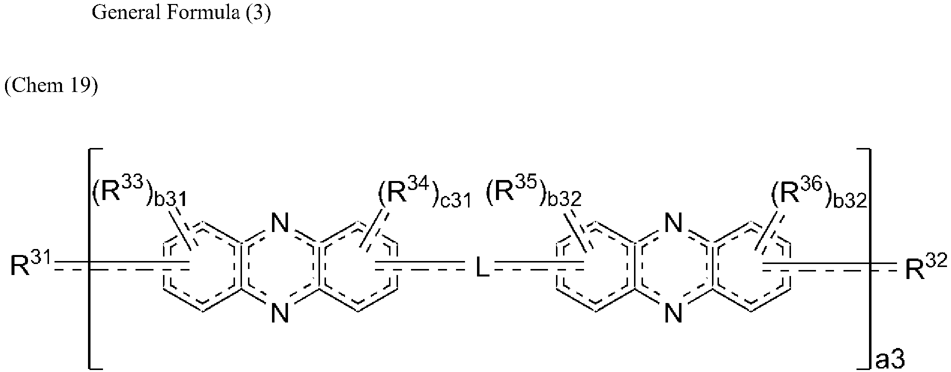

In General Formula (3), the terminal R³¹, the terminal R³², and R³³ to R³⁶ may each independently represent an oxygen atom, a group represented by -OM, a hydrogen atom, or an organic group. M may represent a hydrogen atom, or the monovalent or divalent metal atom. When M is the divalent metal atom, two adjacent ones of R³¹ to R³⁶ may be linked to each other to form a ring represented by -OMO- (R³¹ may be linked to R³³ included in the benzene ring to which R³¹ is bonded, and R³² may be linked to R³⁶ included in the benzene ring to which R³² is bonded). The ring represented by -OMO- may have the same configuration as the -OMO- described above for General Formula (1).

L is a direct bond or the divalent linker that includes an aromatic group, a double bond, or a triple bond. The double line indicated by a solid line and a dashed line indicates the single bond or the double bond. The single bond and the double bond may be arranged such that the compound is the conjugated molecule and has a resonance structure.

In General Formula (3), a3 indicates the number of repeating units of the phenazine structure bonded via the linker described in square brackets. a3 may be the integer from 1 to 5.

In General Formula (3), b31, b32, c31, and c32 may each independently be an integer from 1 to 3. When b31, b32, c31, and c32 are 2 or 3, the two or three R³³, R³⁴, R³⁵, and R³⁶ may each be identical to or different from each other, and at least four of R³³ and R³⁴ and at least four of R³⁵ and R³⁶ may be the oxygen atom or a group represented by -OM. The number of oxygen atoms or OM groups among R³³ and R³⁴ may be the even number of 4 or more. All of R²³ and R²⁴ may be the oxygen atom or the OM group. The number of oxygen atoms or OM groups among R³⁵ and R³⁶ may be the even number of 4 or more. All of R³⁵ and R³⁶ may be the oxygen atom or the OM group.

Each of R³¹ to R³⁶ may have the same configuration as R¹¹ to R¹⁴ described above. For example, R³¹ to R³⁶ each independently represent an oxygen atom, a group represented by -OM, a hydrogen atom, or an organic group. Each of R³¹ to R³⁶ may be determined such that the compound represented by General Formula (3) has symmetry. Each of R³¹ to R³⁶ may be determined such that the compound represented by General Formula (3) has the axis of symmetry, the plane of symmetry, or the center of symmetry.

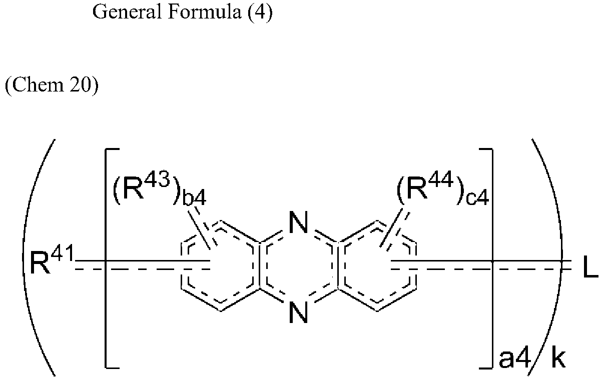

In General Formula (4), terminal R⁴¹, R⁴³, and R⁴⁴ may each independently represent an oxygen atom, a group represented by -OM, a hydrogen atom, or an organic group. M may represent a hydrogen atom, or the monovalent or divalent metal atom. When M is the divalent metal atom, two adjacent ones of R⁴¹, R⁴³, and R⁴⁴ may be linked to each other to form a ring represented by -OMO- (R¹⁴ may be linked to R⁴³ included in the benzene ring to which R⁴¹ is bonded). The ring represented by -OMO- may have the same configuration as the -OMO- described above for the General Formula (1).

L is a direct bond or the k-valent linker that includes an aromatic group, a double bond, or a triple bond. k is the integer of 2 or more. The linker may be an organic group including the arylene group, the heterocyclic group, the alkenylene group, or the alkynylene group. When k is 2, the linker may be the phenylene group, the ethene-1,2-diyl group, or the acetylene-1,2-diyl group. When k is 3 or more, the linker may be, for example, the linker including a benzene ring, the triazine-2,4,6-triyl group, the linker including a phenazine structure, or the like. When k is 3 or more, if the linker has a structure including a nitrogen atom such as triazine or a phenazine structure, steric hindrance is reduced and the active material becomes the molecule with high planarity, thereby improving crystallinity, and thus dissolution of the active material into an electrolytic solution can be further suppressed.

A double line indicated by a solid line and a dashed line indicates the single bond or the double bond. The single bond and the double bond may be arranged such that the compound is the conjugated molecule and has a resonance structure.

In General Formula (4), a4 indicates the number of repeating units of the phenazine structure described in brackets. a4 may be the integer of 1 to 5.

In General Formula (4), b4 and c4 may each independently be the integer of 1 to 3. When b4 and c4 are 2 or 3, two or three R⁴³ and R⁴⁴ may each be identical to or different from each other, and at least four of R⁴³ and R⁴⁴ may be the oxygen atom or a group represented by -OM. The number of oxygen atoms or OM groups among R⁴³ and R⁴⁴ may be the even number of 4 or more. All of R⁴³ and R⁴⁴ may be oxygen atoms or OM groups.

Each of R⁴¹, R⁴³, and R⁴⁴ may have the same configuration as R¹¹ to R¹⁴ described above. For example, R⁴¹, R⁴³, and R⁴⁴ each independently indicate an oxygen atom, a group represented by -OM, a hydrogen atom, or an organic group. Further, each of R⁴¹, R⁴³, and R⁴⁴ may be determined such that the compound represented by General Formula (4) has symmetry. Each of R⁴¹, R⁴³, and R⁴⁴ may be determined such that the compound represented by General Formula (4) has the axis of symmetry, the plane of symmetry, or the center of symmetry.

In this embodiment, the compound represented by General Formula (4) described above may have symmetry. For example, the compound represented by General Formula (4) may have the axis of symmetry, the plane of symmetry, or the center of symmetry.

The above General Formula (1) may be represented by the following General Formula (5). General Formula (5)

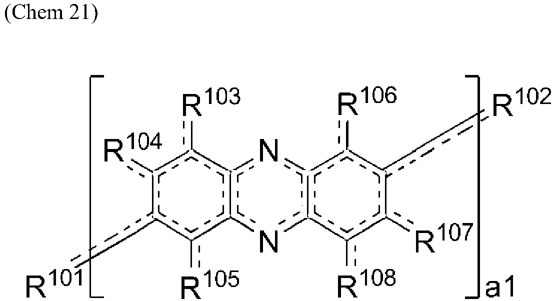

In General Formula (5), terminal R¹⁰¹, terminal R¹⁰², and R¹⁰³ to R¹⁰⁸ may each independently represent an oxygen atom, a group represented by -OM, a hydrogen atom, or an organic group. M may represent a hydrogen atom or the monovalent or divalent metal atom. When M is the divalent metal atom, two adjacent ones of R¹⁰¹ to R¹⁰⁸ may be linked to each other to form a ring represented by -OMO-. The double line indicated by a solid line and a dashed line indicates the single bond or the double bond. At least four of R¹⁰³ to R¹⁰⁸ may be the oxygen atom or a group represented by -OM. The number of oxygen atoms or OM groups among R¹⁰³ to R¹⁰⁸ may be the even number of 4 or more. All of R¹⁰³ to R¹⁰⁸ may be the oxygen atom or the OM group. Each of R¹⁰¹ to R¹⁰⁸ may have the same configuration as R¹¹ to R¹⁴ described above.

In General Formula (5), a1 indicates the number of repeating units of the phenazine structure. a1 may be the integer from 2 to 10.

In this embodiment, the compound represented by General Formula (5) described above may have symmetry. For example, the compound represented by General Formula (5) may have the axis of symmetry, the plane of symmetry, or the center of symmetry.

The General Formula (2) described above may be represented by the following General Formula (6).

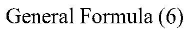

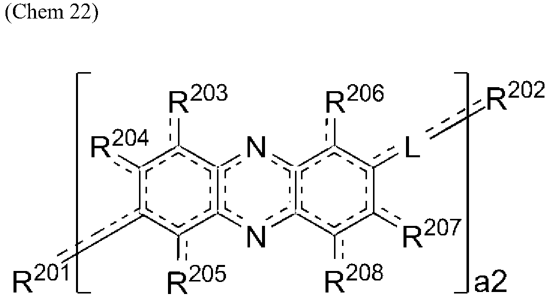

In General Formula (6), terminal R²⁰¹, terminal R²⁰², and R²⁰³ to R²⁰⁸ may each independently represent an oxygen atom, the group represented by -OM, a hydrogen atom, or an organic group. M may represent a hydrogen atom, or the monovalent or divalent metal atom. When M is the divalent metal atom, two adjacent ones of R²⁰¹ to R²⁰⁸ may be linked to each other to form a ring represented by -OMO-. L is a direct bond or the divalent linker including an aromatic group, a double bond, or a triple bond, and may have the same configuration as the linker of General Formula (1) described above. A double line indicated by a solid line and a dashed line indicates the single bond or the double bond. At least four of R²⁰³ to R²⁰⁸ may be the oxygen atom or a group represented by -OM. Each of R²⁰¹ to R²⁰⁸ may have the same configuration as R¹¹ to R¹⁴ described above.

In General Formula (6), a2 indicates the number of repeating units each including a phenazine structure and the linker included in the oligomer. a2 may be the integer from 2 to 10. When a2 is within this range, it is possible to suppress dissolution of the active material into the electrolytic solution during charging and discharging and to improve cycle performance of the secondary battery. In addition, it is possible to suppress the decrease in crystallinity of the active material and the decrease in electronic conductivity.

In this embodiment, the compound represented by the above General Formula (6) may have symmetry. For example, the compound represented by General Formula (6) may have the axis of symmetry, the plane of symmetry, or the center of symmetry.

The above General Formula (3) may be represented by the following General Formula (7). General Formula (7)

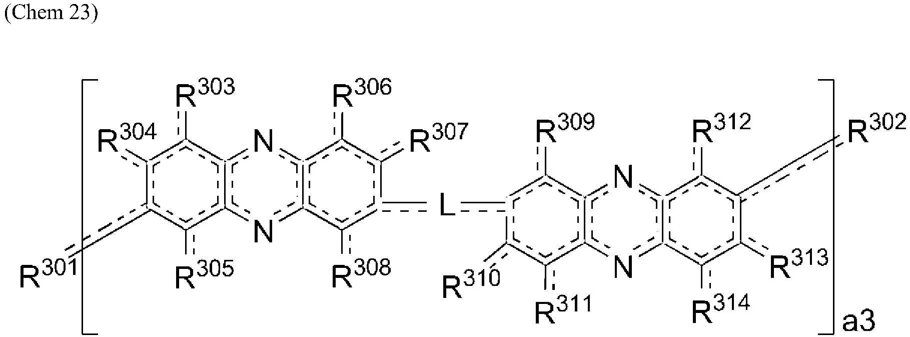

In General Formula (7), terminal R³⁰¹, terminal R³⁰², and R³⁰³ to R³¹⁴ may each independently represent an oxygen atom, the group represented by -OM, a hydrogen atom, or an organic group. M may represent a hydrogen atom, or the monovalent or divalent metal atom. When M is the divalent metal atom, two adjacent ones of R³⁰¹ to R³¹⁴ may be linked to each other to form a ring represented by -OMO-. L is a direct bond or the divalent linker including an aromatic group, a double bond, or a triple bond, and may have the same configuration as the linker of General Formula (1) described above. A double line indicated by a solid line and a dashed line indicates the single bond or the double bond. At least four of R³⁰³ to R³⁰⁸ and at least four of R³⁰⁹ to R³¹⁴ may each be the oxygen atom or a group represented by -OM. Each of R³⁰¹ to R³¹⁴ may have the same configuration as R¹¹ to R¹⁴ described above.

In General Formula (7), a3 indicates the number of repeating units of the phenazine structure bonded via a linker. a3 may be the integer from 1 to 5. When a3 is within this range, it is possible to suppress dissolution of the active material into the electrolytic solution during charging and discharging and to improve cycle performance of the secondary battery. In addition, it is possible to suppress the decrease in crystallinity of the active material and the decrease in electronic conductivity.

In this embodiment, the compound represented by General Formula (7) described above may have symmetry. For example, the compound represented by General Formula (7) may have the axis of symmetry, the plane of symmetry, or the center of symmetry.

General Formula (4) described above may be represented by the following General Formula (8).

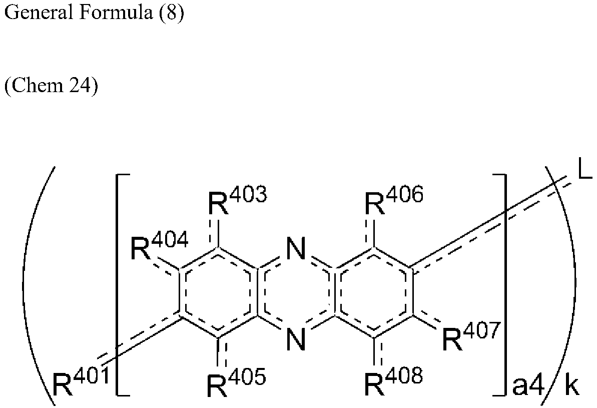

In General Formula (8), terminal R⁴⁰¹ and R⁴⁰³ to R⁴⁰⁸ may each independently represent an oxygen atom, the group represented by -OM, a hydrogen atom, or an organic group. M may represent a hydrogen atom or the monovalent or divalent metal atom. When M is the divalent metal atom, two adjacent R⁴⁰¹ and R⁴⁰³ to R⁴⁰⁸ may be linked to each other to form a ring represented by -OMO-. L is a direct bond or the k-valent linker including an aromatic group, a double bond, or a triple bond, and may have the same configuration as the linker of General Formula (4) described above. k is the integer of 2 or more. The double line indicated by a solid line and a dashed line represents a single bond or the double bond. At least four of R⁴⁰³ to R⁴⁰⁸ may be the oxygen atom or a group represented by -OM.

In General Formula (8), a4 indicates the number of repeating units of the phenazine structure. a4 may be the integer from 1 to 5. When a4 is within this range, dissolution of the active material into the electrolytic solution during charging and discharging can be suppressed, and cycle performance of the secondary battery can be improved. In addition, it is possible to suppress the decrease in crystallinity of the active material and the decrease in electronic conductivity.

In the above General Formulae (1) to (8), L is a direct bond or a divalent or higher-valent linker containing the aromatic group, a double bond, or a triple bond. The linker may be an organic group containing the arylene group, the heterocyclic group, the alkenylene group, or the alkynylene group. The arylene group may be, for example, the phenylene group. The heterocyclic group may include, for example, the phenazine structure.

In General Formula (4), an example in which L includes the phenazine structure is shown in General Formula (9). General Formula (9)

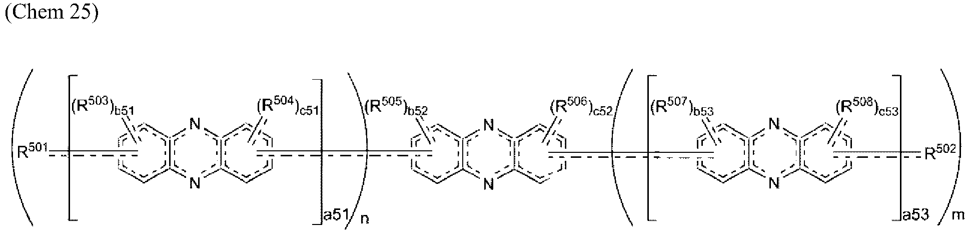

In General Formula (9), terminal R⁵⁰¹, terminal R⁵⁰², and R⁵⁰³ to R⁵⁰⁸ may each independently represent an oxygen atom, the group represented by -OM, a hydrogen atom, or an organic group. M may represent a hydrogen atom, or the monovalent or divalent metal atom. When M is the divalent metal atom, two adjacent ones of R⁵⁰¹ to R⁵⁰⁸ may be linked to each other to form a ring represented by -OMO-. A double line indicated by a solid line and a dashed line indicates the single bond or the double bond. In General Formula (9), by including the phenazine structure in the linker, planarity of the active material including a plurality of phenazine structures is improved, whereby crystallinity of the active material is improved and dissolution into the electrolytic solution can be further suppressed.

In General Formula (9), a51 and a53 may each independently be the integer from 1 to 5. b51, b52, b53, c51, c52, and c53 may each independently be an integer from 1 to 3. When b51, b53, c51, and c53 are 2 or 3, two or three R⁵⁰³, R⁵⁰⁴, R⁵⁰⁷, and R⁵⁰⁸ may each be the same as or different from each other, and at least four of R⁵⁰³ and R⁵⁰⁴ and at least four of R⁵⁰⁷ and R⁵⁰⁸ may be the oxygen atom or a group represented by -OM. n and m may each be the integer from 1 to 3. The sum of n and b52 and the sum of m and c52 may each be 4 or less.

Each of R⁵⁰¹ to R⁵⁰⁸ may have the same configuration as each of the above-described R¹¹ to R¹⁴. For example, R⁵⁰¹ to R⁵⁰⁸ may each independently indicate an oxygen atom, the group represented by -OM, a hydrogen atom, or an organic group. Further, each of R⁵⁰¹ to R⁵⁰⁸ may be determined such that the compound represented by General Formula (9) has symmetry. Each of R⁵⁰¹ to R⁵⁰⁸ may be determined such that the compound represented by General Formula (9) has the axis of symmetry, the plane of symmetry, or the center of symmetry.

### (Specific examples of compounds used as active materials)

In one embodiment, the following compounds are exemplified as specific examples of the above active material.

The following Compound (1) to Compound (5) correspond to a state in which the active material is fully charged. The following compounds may be an example of a fully oxidized form of the compound represented by General Formula (1) to General Formula (9). It should be noted that the following compounds are the conjugated molecules and have resonance structures. The compounds represented by General Formula (1) to General Formula (9) described above are not limited thereto. As other specific examples, the fully reduced form or a partially reduced form of the following compounds may be exemplified.

### (An example of a fully oxidized form)

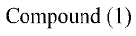

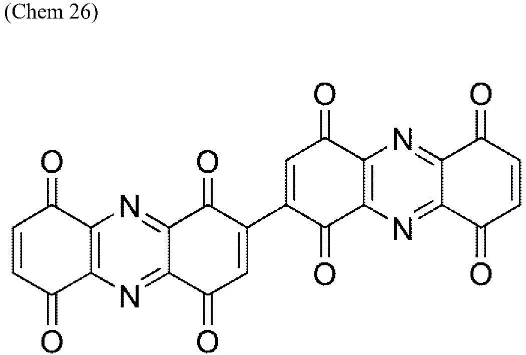

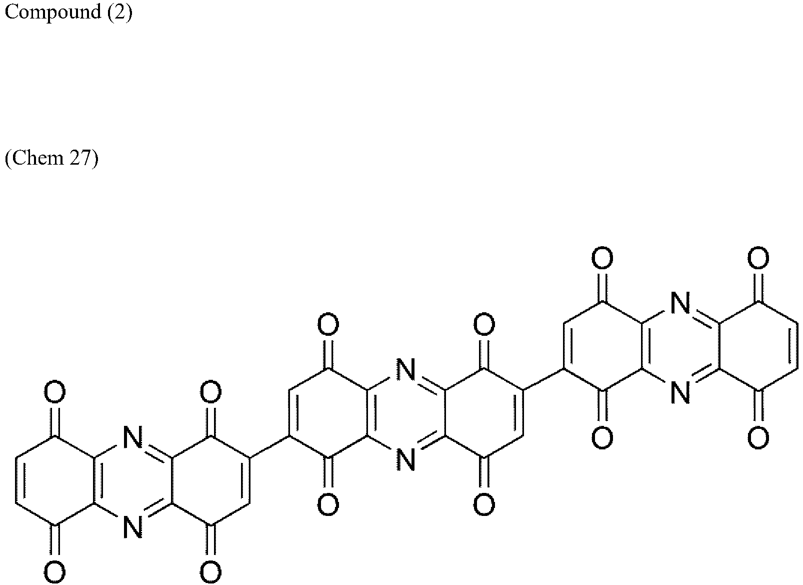

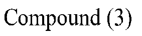

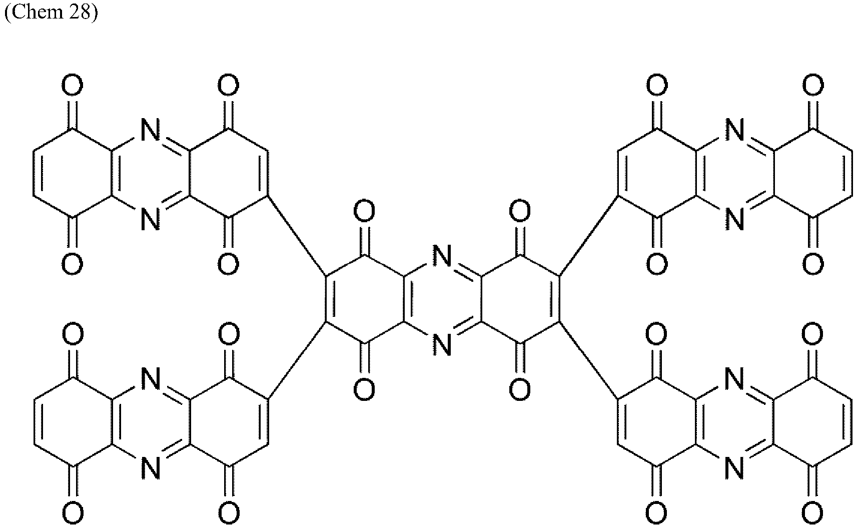

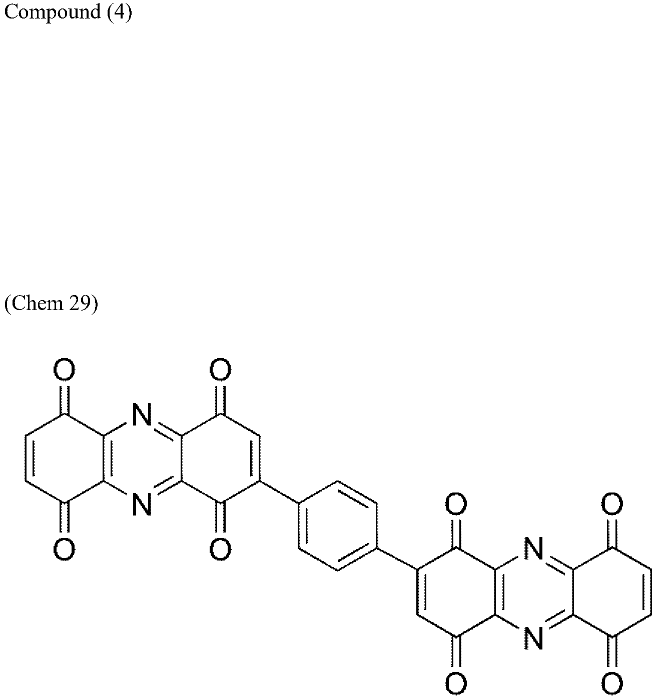

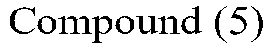

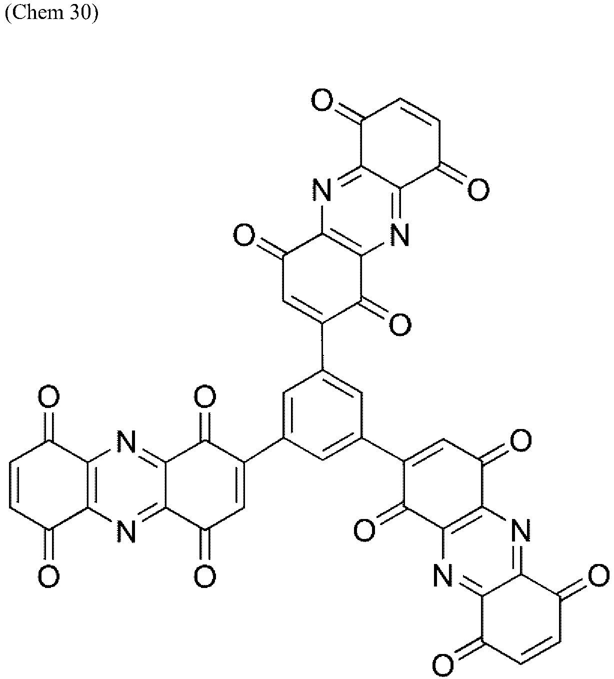

The following compounds (6) to compounds (10) correspond to the state in which the active material is fully discharged. The following compounds (6) to compounds (10) may be an example of the fully reduced form of the compound represented by General Formula (1) to General Formula (9). The following compounds may be the fully reduced form of the above compounds exemplified as compounds corresponding to a state in which the active material is fully charged. It should be noted that the following compounds are the conjugated molecules and have resonance structures.

### (Example of Fully Reduced Form] Compound (6)

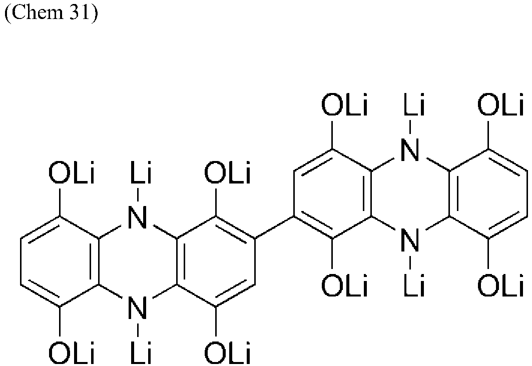

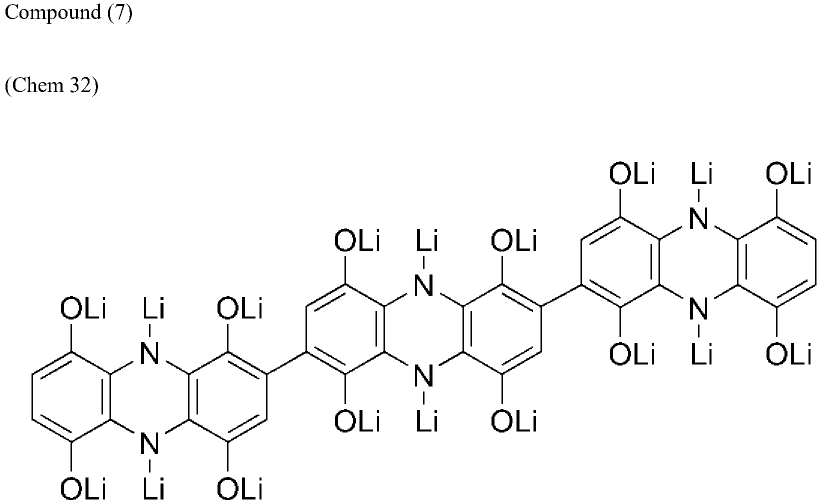

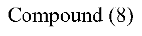

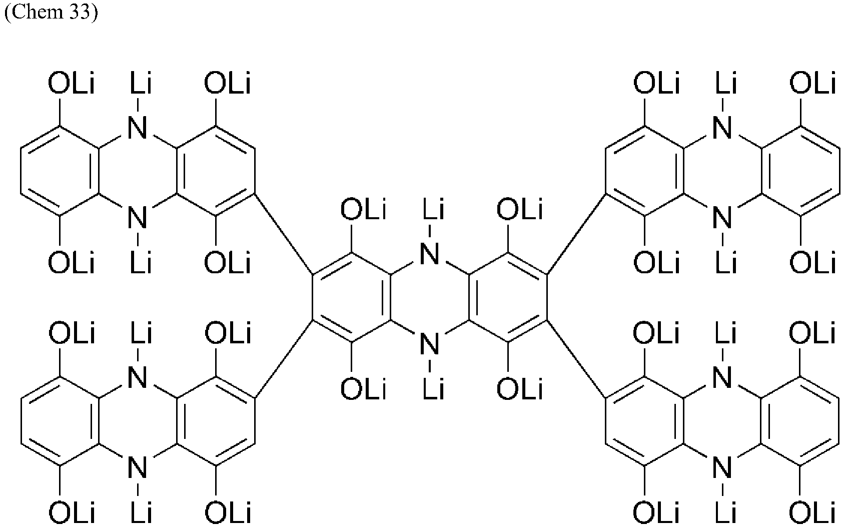

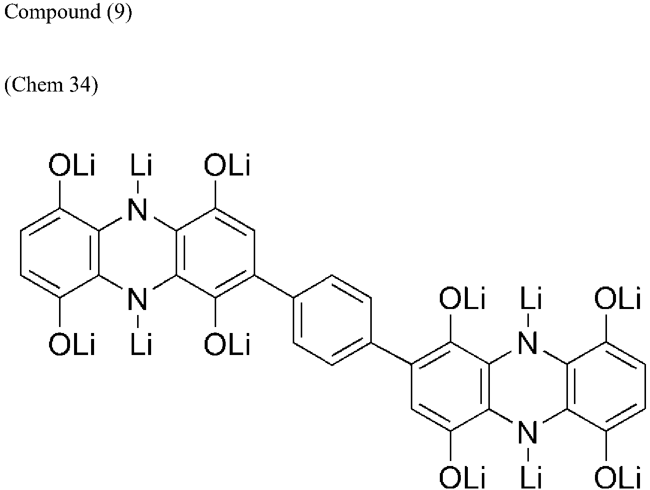

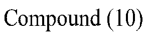

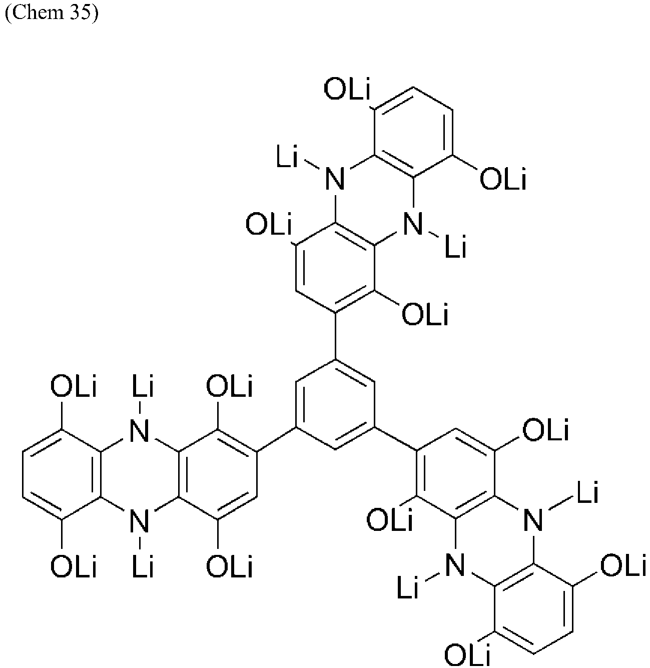

### (Method for producing an active material)

The above active material is the known compound, or can be synthesized by employing a known reaction.

### (Synthesis Example 1)

The dimer oligomer of phenazinetetraone of compound (1) and the trimer oligomer of phenazinetetraone of compound (2) can be synthesized by the following reaction formula (1).

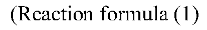

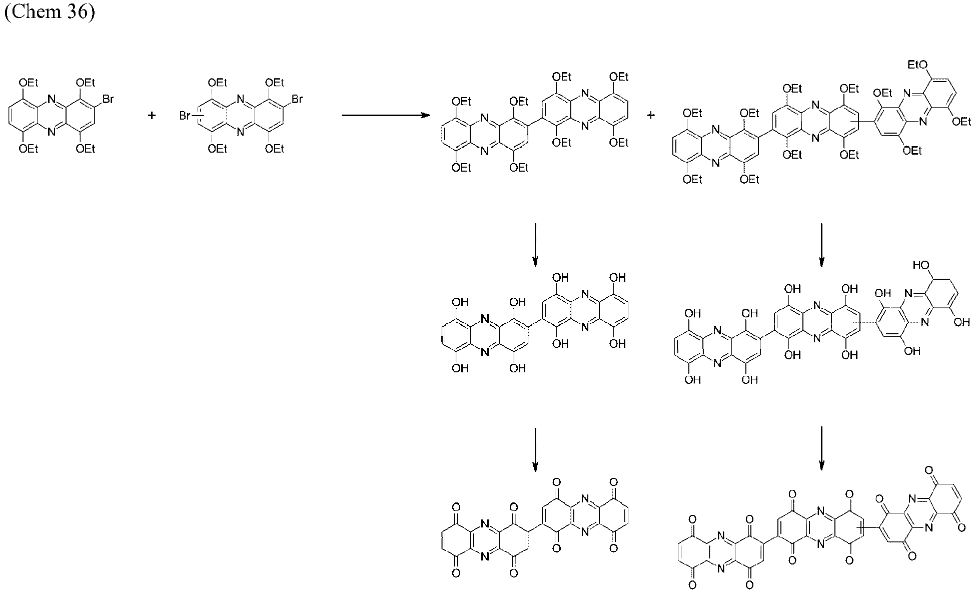

Tetraethoxyphenazine bromide (87.0 mg (0.20 mmol)), tetraethoxyphenazine dibromide (51.4 mg (0.10 mmol)), bis(pinacolato)diboron (63.5 mg (0.25 mmol)), the palladium(0) catalyst (12.5 mol%), and a cesium salt were added to a 100 mL vial. Xylene (5 mL) was added thereto, argon gas was injected, and the vial was sealed. Thereafter, after stirring at 135°C for 24 hours, the solvent was distilled off from the reaction solution, and the obtained yellow solid was dissolved in dichloromethane and crudely purified by the silica gel column to obtain a mixture of a dimer oligomer and a trimer oligomer in which tetraethoxyphenazine was linked by a direct bond. From the oligomer mixture, the dimer of tetraethoxyphenazine and the trimer of tetraethoxyphenazine were respectively fractionated into 50 mL vials by gel permeation chromatography (GPC). Mass spectrometry: 711 ([M(C₄₀H₄₆N₄O₈)+H]⁺), 1065 ([M(C₆₀H₆₈N₆O₁₂)+H]⁺).

Next, to the fractionated dimer of tetraethoxyphenazine, the 25 wt% 1,2-dichloroethane solution in which 71.1 mg (0.100 mmol) and 500 mg of boron tribromide (2.0 mmol) were dissolved was added, followed by heating and stirring at 80°C for 18 hours. After the reaction, pure water was added to the solution to precipitate the dark purple-brown solid. The precipitated solid was collected by filtration and washed with water. The obtained solid was suspended in acetonitrile (10 mL) in the 50 mL vial, the aqueous solution (2 mL) containing CAN (658 mg (1.20 mmol)) was added dropwise, and the mixture was heated and stirred at 40°C for 1 hour. The resulting brown precipitate was collected by filtration and washed with water and methanol. Thereafter, the brown precipitate was air-dried to obtain the brown phenazinetetraone dimer powder. The phenazinetetraone trimer was also synthesized by the same method.

### (Synthesis Example 2)

In addition, the p-phenylenephenazine dimer oligomer in the above compound (4) can be synthesized according to the following reaction formula (2).

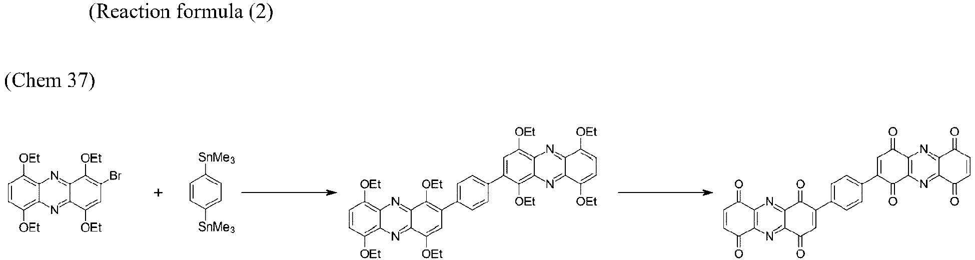

Tetraethoxyphenazine bromide 218 mg (0.5 mmol), 1,4-bis(trimethylstannyl)benzene 80.7 mg (0.2 mmol), and the palladium(0) catalyst 10 mol% were added to a 100 mL vial. This was dissolved in 20 mL of 1,4-dioxane, argon gas was introduced, and the vial was sealed. After heating with stirring at 85°C for 72 hours, the mixture was cooled to room temperature, and the resulting yellow solid was filtered. The collected solid was washed with ethyl acetate and purified by column purification using the silica gel to obtain a phenylene dimer of tetraethoxyphenazine as a yellow solid. Mass spectrometry: 787 ([M(C₄₆H₅₀N₄O₈)+H]⁺).

Of the obtained yellow solid described above, 78.7 mg (0.10 mmol) was added to the 100 mL vial and suspended in 10 mL of acetonitrile. The suspension was stirred at room temperature, the aqueous solution (2 mL) containing 658 mg (1.2 mmol) of ammonium cerium(IV) hexanitratocerate (hereinafter, CAN) was added dropwise, and the mixture was stirred at room temperature for 2 hours. The yellowish-brown precipitate formed in the solution was collected by filtration and washed with water and methanol. After air-drying, the phenylene dimer of phenazine tetron was obtained as a brown solid.

### (Example)

The R2032 coin-type battery was produced using, as a positive electrode active material, the phenazines synthesized in Synthesis Example 1 and Synthesis Example 2 described above. The R2032 coin-type battery was produced by the following procedure.

First, phenazines as a positive electrode active material, acetylene black (manufactured by Denka Company Limited) as the conductive additive, and PTFE (manufactured by Daikin Industries, Ltd.) as a binding agent were mixed at a ratio of positive electrode active material:conductive additive:binding agent = 4:5:1 (mass ratio) to produce a positive electrode active material sheet having a diameter of about 10 mm and a thickness of about 100 µm. A positive electrode was produced by pressing the positive electrode active material sheet described above to the stainless steel mesh (manufactured by Hosen Co., Ltd., SUS316L) having a diameter of 14 mm and a thickness of 100 µm.

Next, the circular member having a diameter of 13 mm was cut out from a metallic lithium foil having a thickness of 0.5 mm (manufactured by Honjo Metal Co., Ltd., purity of 99.8% or more). A negative electrode was produced by pressing the above member cut out from the above lithium foil onto the stainless steel plate having a diameter of 15.5 mm and a thickness of 0.5 mm (manufactured by Hosen Co., Ltd.).

Further, as the separator, the glass filter having a diameter of 16 mm and a thickness of 0.4 mm (manufactured by Advantech Co., Ltd.) was prepared. As the electrolytic solution, the nonaqueous electrolytic solution (manufactured by Kishida Chemical Co., Ltd.) containing LiPF₆ and a mixture of ethylene carbonate and diethyl carbonate was prepared. A test coin-type battery was produced by disposing the above positive electrode, separator, negative electrode, and electrolytic solution inside the battery case compliant with the standard of an R2032 coin-type battery.

For the produced test coin-type battery, the charge-discharge test was performed under an atmosphere at 30°C at a current density of 20 mA/g in a voltage range of 1.2-3.5 V (vs. Li_{C.E.}) or 1.0-4.2 V (vs. Li_{C.E.}).

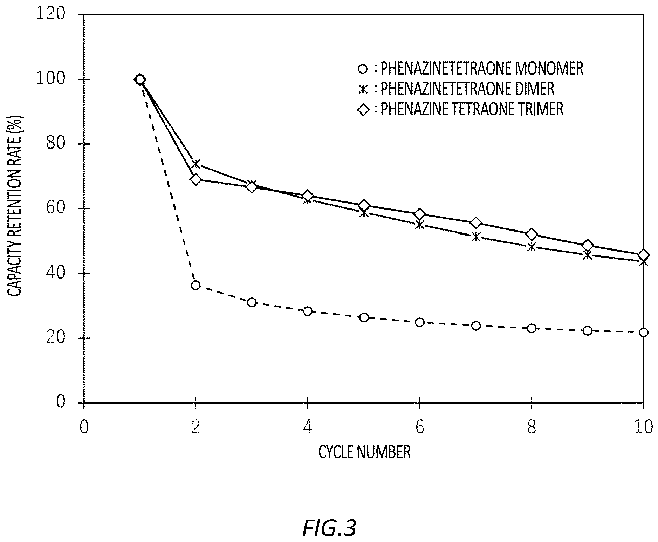

FIG. 3 shows changes in capacity retention rate in the charge-discharge test of the secondary battery of this embodiment. In FIG. 3, transitions with cycles of capacity retention rates of secondary batteries produced using, as a positive electrode active material, the dimer oligomer of phenazinetetraone of compound (1), the trimer oligomer of phenazinetetraone of compound (2), and, as a comparative example, the monomer of phenazinetetraone are shown. The discharge capacity in the first cycle was 363 mAh/g when the dimer oligomer of phenazinetetraone was used, and exhibited a high capacity of 442 mAh/g when the trimer oligomer of phenazinetetraone was used. In the second cycle of the charge-discharge test, the capacity retention rates of the secondary batteries using the dimer oligomer and the trimer oligomer became higher than the capacity retention rate of the secondary battery using the monomer. The capacity after 10 cycles was 159 mAh/g in the case of the dimer oligomer and 203 mAh/g in the case of the trimer oligomer, and exhibited a higher discharge capacity than the monomer. The capacity retention rate after 10 cycles was 44% for the dimer oligomer and 46% for the trimer oligomer, and exhibited a higher capacity retention rate than the monomer. Therefore, the secondary battery including, as an active material, an oligomer in which a plurality of phenazine structures are bonded can maintain a higher capacity retention rate than monomeric phenazines. By using the secondary battery active material according to this embodiment as the active material, it is possible to provide a secondary battery in which the amount of electric charge that can be stored per unit mass is large and cycle performance is improved.

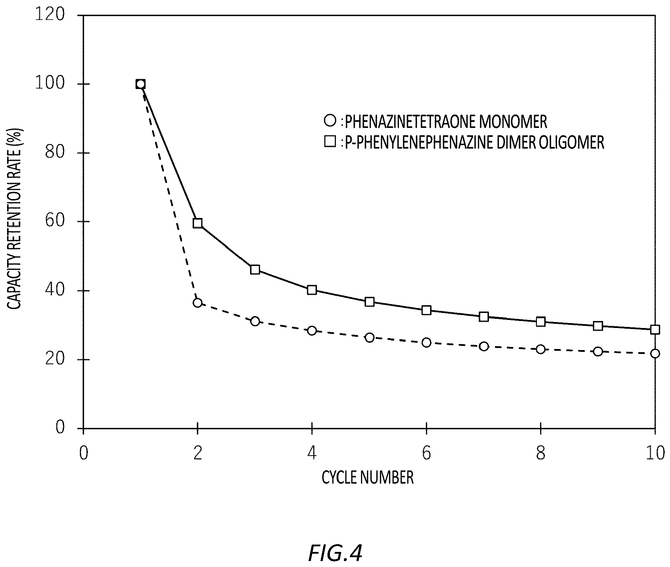

FIG. 4 is a diagram illustrating changes in the capacity retention rate in a charge-discharge test of the secondary battery of this embodiment. FIG. 4 represents changes with cycles in capacity retention rate of a secondary battery produced using the p-phenylenephenazine dimer oligomer of compound (4) and, as a comparative example, the phenazinetetraone monomer as an active material. In the secondary battery using the p-phenylenephenazine dimer oligomer, the discharge capacity in the first cycle exhibited a high discharge capacity of 439 mAh/g. Further, in the secondary battery using the p-phenylenephenazine dimer oligomer, the capacity decrease with cycles was smaller than in the case of using the phenazinetetraone monomer, and in the second cycle, it exhibited a higher capacity retention rate than in the case of using the phenazinetetraone monomer. When the p-phenylenephenazine dimer oligomer was used, the discharge capacity after 10 cycles was 126 mAh/g, and the capacity retention rate was 29%. On the other hand, in the phenazinetetraone monomer, the discharge capacity after 10 cycles was 112 mAh/g, and the capacity retention rate was 22%. Therefore, the secondary battery including, as an active material, an oligomer in which a plurality of phenazine structures are bonded via a linker can maintain a higher capacity retention rate than monomeric phenazines. By using the secondary battery active material according to this embodiment as an active material, it is possible to provide the secondary battery in which the amount of electric charge that can be stored per unit mass is large and cycle performance is improved.

Although the present invention has been described above using embodiments, the technical scope of the present invention is not limited to the scope described in the above embodiments. It is apparent to those skilled in the art that various modifications or improvements can be made to the above embodiments. It is apparent from the description of the claims that embodiments to which such modifications or improvements are added can also be included in the technical scope of the present invention.

It should be noted that the execution order of the processes such as operations, procedures, steps, and stages in the apparatus, system, program, and method indicated in the claims, the specification, and the drawings is not expressly specified as being "before," "prior to," or the like, and can be implemented in any order unless the output of a preceding process is used in a subsequent process. With respect to the operation flows in the claims, the specification, and the drawings, even if they are described using, for convenience, "first," "next," or the like, this does not mean that implementation in this order is essential.

### EXPLANATION OF REFERENCES

100 flight vehicle, 110 storage battery, 112 storage cell, 120 power control circuit, 130 electric motor, 140 propeller, 150 sensor, 160 controller, 212 positive electrode case, 214 negative electrode case, 216 sealant, 218 metal spring, 220 positive electrode, 222 positive electrode current collector, 224 positive electrode active material layer, 230 separator, 240 negative electrode, 242 negative electrode current collector, 244 negative electrode active material layer, 250 electrolytic solution.

## Claims

1. A secondary battery active material used as an active material for a secondary battery, wherein the active material includes at least any compound represented by following General Formula (1), General Formula (2), General Formula (3), and General Formula (4), or a salt thereof, wherein
General Formula (1) is: 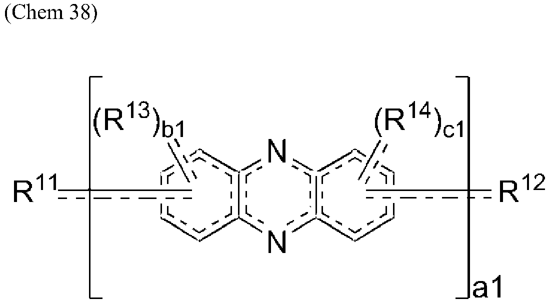
in General Formula (1), terminal R¹¹, terminal R¹², R¹³, and R¹⁴ each independently represent an oxygen atom, a group represented by -OM, a hydrogen atom, or an organic group, M represents a hydrogen atom or a monovalent or divalent metal atom, and when M is a divalent metal atom, two adjacent ones of R¹¹ to R¹⁴ are linked to each other to form a ring represented by -OMO-,
double lines indicated by solid lines and dashed lines represent a single bond or a double bond,
a1 is an integer from 2 to 10, b1 and c1 are each independently an integer from 1 to 3, and
when b1 and c1 are 2 or 3, two or three R¹³ and R¹⁴ are each the same as or different from each other, and at least four of R¹³ and R¹⁴ are an oxygen atom or a group represented by -OM,
General Formula (2) is: 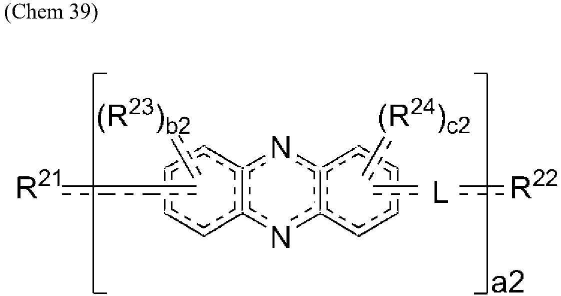
in General Formula (2), terminal R²¹, terminal R²², R²³, and R²⁴ each independently represent an oxygen atom, a group represented by -OM, a hydrogen atom, or an organic group, M represents a hydrogen atom or a monovalent or divalent metal atom, and when M is a divalent metal atom, two adjacent ones of R²¹ to R²⁴ are linked to each other to form a ring represented by -OMO-,
L is a direct bond or a divalent linker including an aromatic group, a double bond, or a triple bond,
double lines indicated by solid lines and dashed lines represent a single bond or a double bond,
a2 is an integer from 2 to 10, b2 and c2 are each independently an integer from 1 to 3, and
when b2 and c2 are 2 or 3, two or three R²³ and R²⁴ are each the same as or different from each other, and at least four of R²³ and R²⁴ are an oxygen atom or a group represented by -OM,
General Formula (3) is: 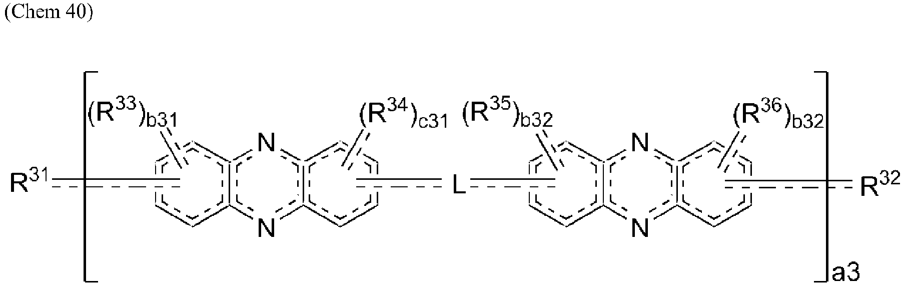
in General Formula (3), terminal R³¹, terminal R³², and R³³ to R³⁶ each independently represent an oxygen atom, a group represented by -OM, a hydrogen atom, or an organic group, M represents a hydrogen atom or a monovalent or divalent metal atom, and when M is a divalent metal atom, two adjacent ones of R³¹ to R³⁶ are linked to each other to form a ring represented by -OMO-,
L is a direct bond or a divalent linker including an aromatic group, a double bond, or a triple bond,
double lines indicated by solid lines and dashed lines represent a single bond or a double bond,
a3 is an integer from 1 to 5, b31, b32, c31, and c32 are each independently an integer from 1 to 3, and when b31, b32, c31, and c32 are 2 or 3, two or three R³³, R³⁴, R³⁵, and R³⁶ are each the same as or different from each other, and at least four of R³³ and R³⁴ and at least four of R³⁵ and R³⁶ are an oxygen atom or a group represented by -OM,
General Formula (4) is: 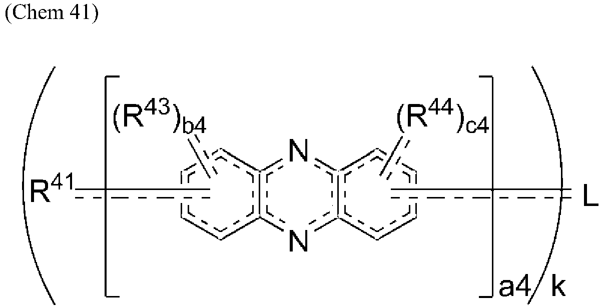
in General Formula (4), terminal R⁴¹, R⁴³, and R⁴⁴ each independently represent an oxygen atom, a group represented by -OM, a hydrogen atom, or an organic group, M represents a hydrogen atom or a monovalent or divalent metal atom, and when M is a divalent metal atom, two adjacent ones of R⁴¹, R⁴³, and R⁴⁴ are linked to each other to form a ring represented by -OMO-,
L is a direct bond or a k-valent linker including an aromatic group, a double bond, or a triple bond,
double lines indicated by solid lines and dashed lines represent a single bond or a double bond,
a4 is an integer from 1 to 5, b4 and c4 are each independently an integer from 1 to 3,
when b4 and c4 are 2 or 3, two or three R⁴³ and R⁴⁴ are each the same as or different from each other, at least four of R⁴³ and R⁴⁴ are an oxygen atom or a group represented by -OM, and
k is an integer of 2 or more.

2. The secondary battery active material according to claim 1, wherein the compound is a compound represented by following General Formula (5) to General Formula (8), wherein.
General Formula (5) is: 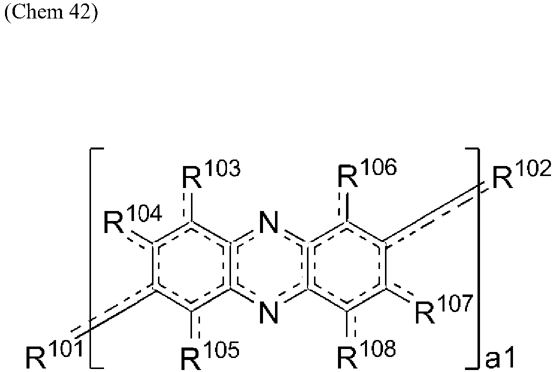
in General Formula (5), terminal R¹⁰¹, terminal R¹⁰², and R¹⁰³ to R¹⁰⁸ each independently represent an oxygen atom, a group represented by -OM, a hydrogen atom, or an organic group, M represents a hydrogen atom or a monovalent or divalent metal atom, and when M is a divalent metal atom, two adjacent ones of R¹⁰¹ to R¹⁰⁸ are linked to each other to form a ring represented by -OMO-,
double lines indicated by solid lines and dashed lines represent a single bond or a double bond,
at least four of R¹⁰³ to R¹⁰⁸ are an oxygen atom or a group represented by -OM, and
a1 is an integer from 2 to 10,
General Formula (6) is: 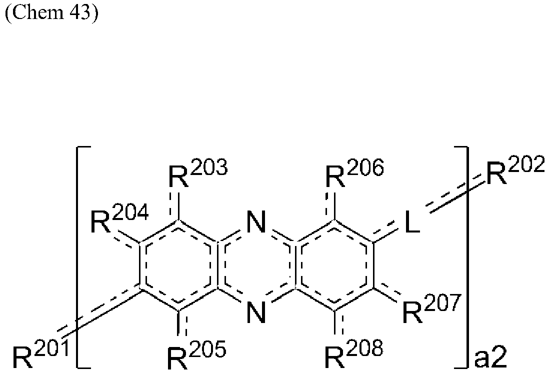
in General Formula (6), terminal R²⁰¹, terminal R²⁰², and R²⁰³ to R²⁰⁸ each independently represent an oxygen atom, a group represented by -OM, a hydrogen atom, or an organic group, M represents a hydrogen atom or a monovalent or divalent metal atom, and when M is a divalent metal atom, two adjacent ones of R²⁰¹ to R²⁰⁸ are linked to each other to form a ring represented by -OMO-,
L is a direct bond or a divalent linker including an aromatic group, a double bond, or a triple bond,
double lines indicated by solid lines and dashed lines represent a single bond or a double bond,
at least four of R²⁰³ to R²⁰⁸ are an oxygen atom or a group represented by -OM, and
a2 is an integer from 2 to 10,
General Formula (7) is: 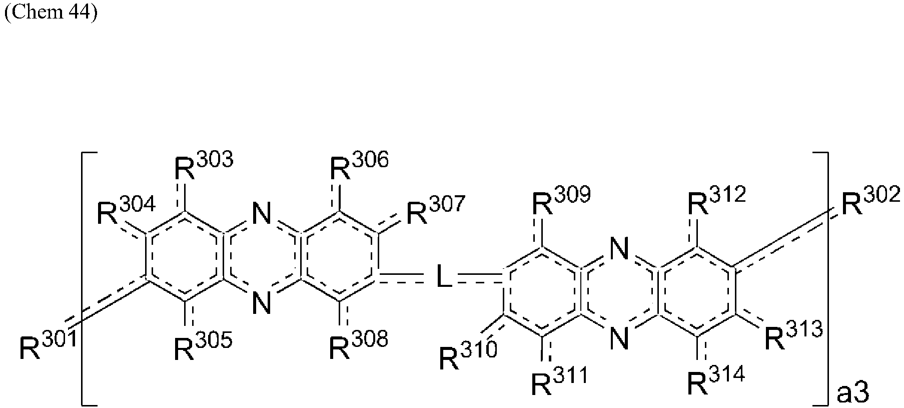
in General Formula (7), terminal R³⁰¹, terminal R³⁰², and R³⁰³ to R³¹⁴ each independently represent an oxygen atom, a group represented by -OM, a hydrogen atom, or an organic group, M represents a hydrogen atom or a monovalent or divalent metal atom, and when M is a divalent metal atom, two adjacent ones of R³⁰¹ to R³¹⁴ are linked to each other to form a ring represented by -OMO-,
L is a direct bond or a divalent linker including an aromatic group, a double bond, or a triple bond,
double lines indicated by solid lines and dashed lines represent a single bond or a double bond,
at least four of R³⁰³ to R³⁰⁸ and at least four of R³⁰⁹ to R³¹⁴ are an oxygen atom or a group represented by -OM, and
a3 is an integer from 1 to 5,
General Formula (8) is: 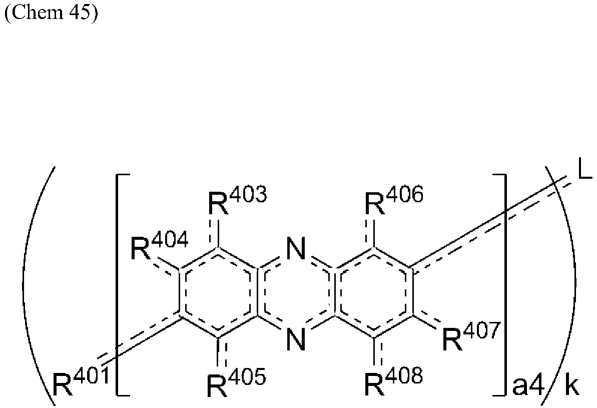
in General Formula (8), terminal R⁴⁰¹ and R⁴⁰³ to R⁴⁰⁸ each independently represent an oxygen atom, a group represented by -OM, a hydrogen atom, or an organic group, M represents a hydrogen atom or a monovalent or divalent metal atom, and when M is a divalent metal atom, two adjacent ones of R⁴⁰¹ and R⁴⁰³ to R⁴⁰⁸ are linked to each other to form a ring represented by -OMO-,
L is a direct bond or a k-valent linker including an aromatic group, a double bond, or a triple bond,
double lines indicated by solid lines and dashed lines represent a single bond or a double bond,
at least four of R⁴⁰³ to R⁴⁰⁸ are an oxygen atom or a group represented by -OM,
a4 is an integer from 1 to 5, and
k is an integer of 2 or more.

3. The secondary battery active material according to claim 1, wherein the compound is a compound represented by following General Formula (9): 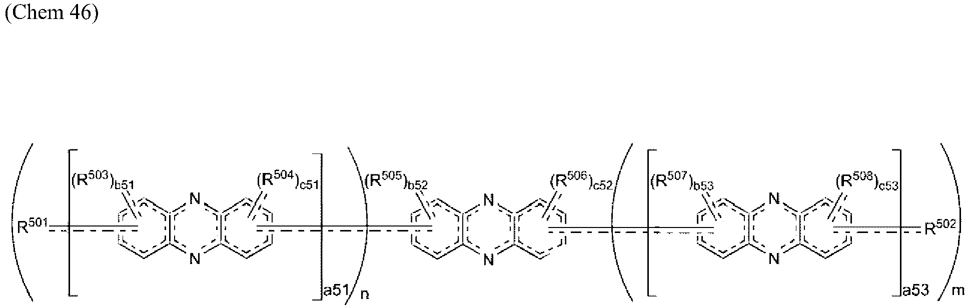
in General Formula (9), terminal R⁵⁰¹, terminal R⁵⁰², and R⁵⁰³ to R⁵⁰⁸ each independently represent an oxygen atom, a group represented by -OM, a hydrogen atom, or an organic group, M represents a hydrogen atom or a monovalent or divalent metal atom, and when M is a divalent metal atom, two adjacent ones of R⁵⁰¹ to R⁵⁰⁸ are linked to each other to form a ring represented by -OMO-,
double lines indicated by solid lines and dashed lines represent a single bond or a double bond,
a51 and a53 are each independently an integer from 1 to 5, b51, b52, b53, c51, c52, and c53 are each independently an integer from 1 to 3,
when b51, b53, c51, and c53 are 2 or 3, two or three R⁵⁰³, R⁵⁰⁴, R⁵⁰⁷, and R⁵⁰⁸ are each the same as or different from each other, and at least four of R⁵⁰³ and R⁵⁰⁴ and at least four of R⁵⁰⁷ and R⁵⁰⁸ are an oxygen atom or a group represented by -OM,
n and m are each an integer from 1 to 3, and
a sum of n and b52 and a sum of m and c52 are each 4 or less.

4. The secondary battery active material according to any one of claims 1 to 3, wherein the compound has symmetry.

5. The secondary battery active material according to any one of claims 1, 2, and 4, wherein the L is a direct bond or an organic group including an arylene group, a heterocyclic group, an alkenylene group, or an alkynylene group.

6. The secondary battery active material according to claim 1, wherein the compound is at least any one of 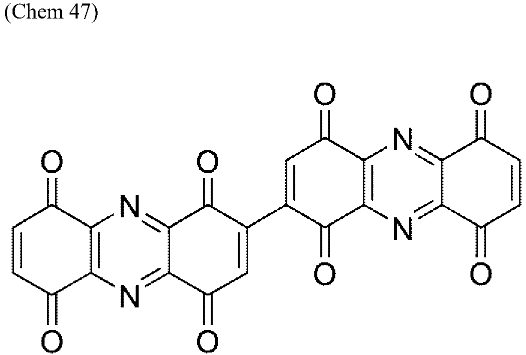 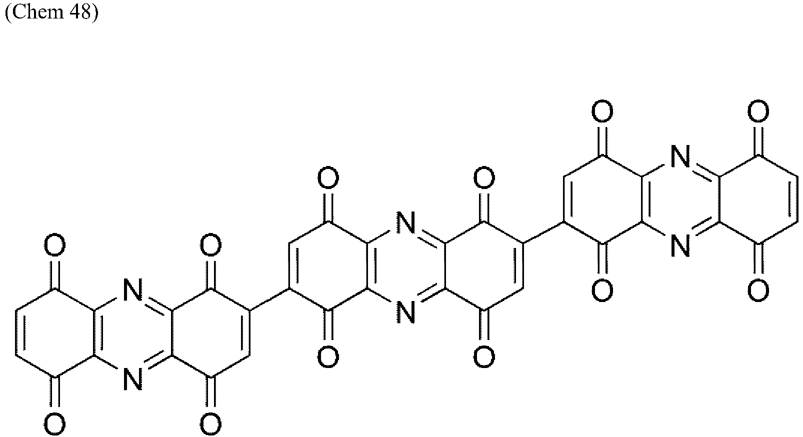 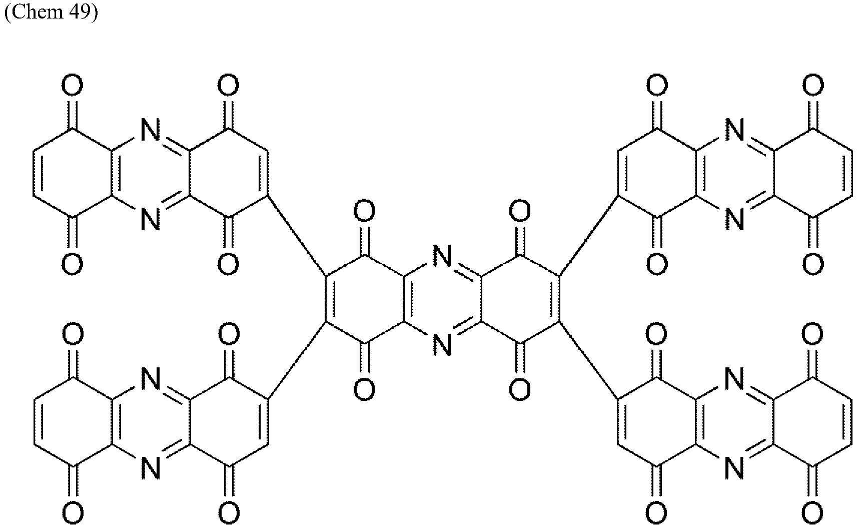 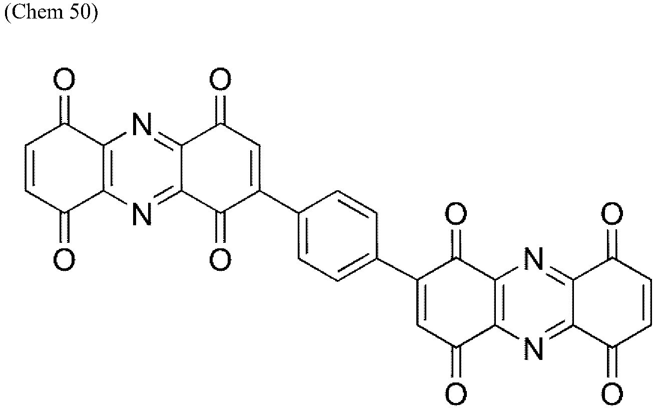 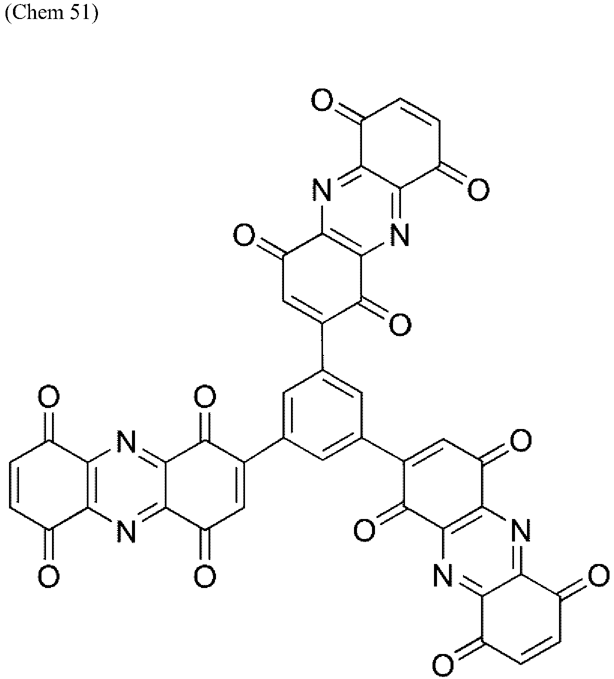 , or a fully reduced form or a partially reduced form thereof.

7. The secondary battery active material according to any one of claims 1 to 6, wherein M is H, Li, Na, K, Mg, or Ca.

8. A secondary battery electrode comprising the secondary battery active material according to any one of claims 1 to 7.

9. A secondary battery comprising:
a positive electrode active material layer containing a positive electrode active material;
a negative electrode active material layer containing a negative electrode active material; and
an electrolyte,
wherein the positive electrode active material or the negative electrode active material contains the secondary battery active material according to any one of claims 1 to 7.

10. The secondary battery according to claim 9, wherein the positive electrode active material contains the secondary battery active material according to claim 1.]

11. The secondary battery according to claim 9 or 10, wherein the secondary battery is a nonaqueous secondary battery.

12. A flight vehicle comprising:
the secondary battery according to any one of claims 9 to 11; and
a propulsive force generator that generates propulsive force by using electrical energy stored in the secondary battery.
